(19) **Europäisches Patentamt / European Patent Office / Office européen des brevets**

(11) **EP 4 292 617 B1**

(12) **EUROPEAN PATENT SPECIFICATION**

(45) Date of publication and mention
of the grant of the patent:
**29.01.2025 Bulletin 2025/05**

(21) Application number: **22179834.1**

(22) Date of filing: **20.06.2022**

(51) International Patent Classification (IPC):
*A61L 27/20* (2006.01)   *A61L 27/38* (2006.01)
*A61L 27/48* (2006.01)   *A61L 27/50* (2006.01)
*A61L 27/52* (2006.01)   *A61L 27/54* (2006.01)
*B33Y 10/00* (2015.01)   *B33Y 70/00* (2020.01)

(52) Cooperative Patent Classification (CPC):
(C-Sets available)
**A61L 27/48; A61L 27/20; A61L 27/38; A61L 27/50;
A61L 27/52; A61L 27/54; B33Y 10/00; B33Y 70/00;**
A61L 2300/41; A61L 2300/416         (Cont.)

(54) **COMPOSITION FOR ADDITIVE MANUFACTURING COMPRISING A COMPOSITE HYDROGEL, PROCESS FOR OBTAINING SAID COMPOSITION, AND PROCESS OF ADDITIVE MANUFACTURING OF AN OBJECT USING SAID COMPOSITION**

ZUSAMMENSETZUNG ZUR GENERATIVEN FERTIGUNG MIT EINEM ZUSAMMENGESETZTEN HYDROGEL, VERFAHREN ZU DEREN HERSTELLUNG UND VERFAHREN ZUR GENERATIVEN FERTIGUNG EINES GEGENSTANDES UNTER VERWENDUNG DIESER ZUSAMMENSETZUNG

COMPOSITION DE FABRICATION ADDITIVE COMPRENANT UN HYDROGEL COMPOSITE, PROCÉDÉ D'OBTENTION DE LADITE COMPOSITION ET PROCÉDÉ DE FABRICATION ADDITIVE D'UN OBJET UTILISANT LADITE COMPOSITION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2022 PT 2022118047**

(43) Date of publication of application:
**20.12.2023 Bulletin 2023/51**

(73) Proprietor: **Universidade de Aveiro
3810-193 Aveiro (PT)**

(72) Inventors:
• **DA ROCHA FREIRE BARROS, CARMEN SOFIA
3840-341 SOSA (PT)**
• **CUNHA VILELA, CARLA ANDREIA
3810-928 AVEIRO (PT)**
• **DOMINGUES SILVESTRE, ARMANDO JORGE
3800-013 AVEIRO (PT)**
• **FERNANDES CARVALHO, JOÃO PEDRO
4615-673 LIXA (PT)**
• **MARTINHO MARQUES DE OLIVEIRA, JOSÉ
3860-051 AVANCA (PT)**

(74) Representative: **Monteiro Alves, Inês
Alameda Dos Oceanos, Nº 41K-21
Parque das Nações
1990-207 Lisboa (PT)**

(56) References cited:
• **YOON HYUNG SUN ET AL: "Cellulose nanocrystals as support nanomaterials for dual droplet-based freeform 3D printing",** CARBOHYDRATE POLYMERS, vol. 272, no. 118459, 22 July 2021 (2021-07-22), GB, pages 1 - 10, XP055949466, ISSN: 0144-8617, DOI: 10.1016/ j.carbpol.2021.118469
• **YAN HUIQIONG ET AL: "Layer-by-layer assembly of 3D alginate-chitosan-gelatin composite scaffold incorporating bacterial cellulose nanocrystals for bone tissue engineering",** MATERIALS LETTERS, vol. 209, 24 August 2017 (2017-08-24), pages 492 - 496, XP085204917, ISSN: 0167-577X, DOI: 10.1016/ J.MATLET.2017.08.093

**(Cont. next page)**

(52) Cooperative Patent Classification (CPC): (Cont.)

C-Sets
**A61L 27/48, C08L 5/04**

**Description**

## TECHNICAL FIELD

The present invention refers to a composition for additive manufacturing comprising a composite hydrogel, a process of obtaining said composition, and an additive manufacture process of an object, wherein the object may be used in several applications, for instance an object referred to a biomedical device.

## BACKGROUND ART

The impact of additive manufacturing, namely 3D printing, in different scientific and technological fields is undeniable. The possibility of fabricating complex structures with precise shape, using the computer-controlled layer-by-layer deposition of materials, has opened the doors for new opportunities in multiple domains, and the pharmaceutical and biomedical sectors are no exception. 3D printing technology may be used in the development of new drug-delivery systems, other biomedical devices, and even in the manufacturing of artificial living tissues. In this latter field, known as 3D bioprinting (one of the younger siblings of 3D printing techniques), it is possible to create living constructs with customized shapes and characteristics, for application in organ transplantation, regenerative medicine, or even drug development.

The materials that are dispensed by the 3D printers are known as inks, and they are a crucial element for the success of the procedure. Therefore, they must be carefully selected considering the printing technique and the desired application. Specifically, the inks should fulfil the physical requirements (and especially the rheological properties) of the 3D printing process. When they are developed for biological endeavors, these materials should also be friendly for living cells, exhibiting biocompatibility and, in some cases, biodegradable nature. In bioprinting, for instance, bioinks containing cells should be able to satisfy the essential conditions for cells to survive the bioprinting procedure, and to proliferate in the newly formed tissue.

There are several 3D printing approaches, but extrusion 3D printing is one of the most explored. This 3D printing technique is based on the application of mechanical or pneumatic pressure forces to promote the ejection of the ink through a nozzle and its deposition. Hydrogel-based inks are one of the most explored biomaterials, and they are frequently applied in extrusion-based processes, as it is disclosed in Schwab, A. et al. Printability and Shape Fidelity of Bioinks in 3D Bioprinting. Chem. Rev. 120, 11028-11055 (2020). Hydrogels are polymeric networks that are able to contain large amounts of water, and closely mimic the native 3D microenvironment of living tissues, granting them with great potential for biological endeavors, as it is disclosed in Unagolla, J. M. & Jayasuriya, A. C. Hydrogel-based 3D bioprinting: A comprehensive review on cell-laden hydrogels, bioink formulations, and future perspectives. Appl. Mater. Today 18, 100479 (2020). They may be obtained from different polymers, including synthetic options like poly(ethylene glycol) (PEG) and polycaprolactone (PCL), according to Kumar, S., Tharayil, A. & Thomas, S. 3D Bioprinting of Nature-Inspired Hydrogel Inks Based on Synthetic Polymers. ACS Appl. Polym. Mater. 3, 3685-3701 (2021); or biopolymers like sodium alginate, according to Datta, S., Barua, R. & Das, J. Importance of Alginate Bioink for 3D Bioprinting in Tissue Engineering and Regenerative Medicine. in Alginates - Recent Uses of This Natural Polymer (IntechOpen, 2020). doi:10.5772/intechopen.90426; chitosan, according to Taghizadeh, M. et al. Chitosan-based inks for 3D printing and bioprinting. Green Chem. 24, 62-101 (2022); or gelatin, according to Wang, X. et al. Gelatin-Based Hydrogels for Organ 3D Bioprinting. Polymers (Basel). 9, 401 (2017).

The use of PEG, for instance, is shown in patent document WO2021234141A1, Guasch Camell Judit et al, published on November 25th, 2021, where the inventors describe a hydrogel from PEG combined with heparin and an immune molecule (i.e., a cytokine or cell-adhesion molecule) for T-cells culture and immunotherapy applications. On the other hand, the use of biopolymers like sodium alginate or gelatin is reported in patent documents like WO2019173637A1, Nelson Kimberly et al, published on September 12th, 2019, concerning an alginate bioink containing nanocelluloses for 3D bioprinting, and in patent document WO2020081982A1, Ogle Brenda M et al, published on April 23rd, 2020, where the authors describe, for instance, a bioink comprising gelatin methacrylate, collagen methacrylate and a photoinitiator for 3D bioprinting of cardiomyocytes.

Yoon Hyung Sun et al. "Cellulose nanocrystals as support nanomaterials for dual droplet-based freeform 3D printing", CARBOHYDRATE POLYMERS vol. 272, no. 118459, 22 July 2021, discloses a composition for additive manufacturing comprising a composite hydrogel comprising an alginate and cellulose nanocrystals, wherein said cellulose nanocrystals are stiff and spindle-shaped crystalline nanomaterials having high aspect ratios (length 50-500 nm, width 3-5 nm).

There is a clear research tendency to focus on natural polymers, also designated as biopolymers, for health-related applications, given their superior biological performance. Among the panoply of natural polymers, sodium alginate, from here on referred to as alginate, is one of the most widely explored biopolymer for 3D printing, envisioning the fabrication of constructs for pharmaceutical and biomedical purposes. Alginate is a linear anionic polysaccharide obtained from brown algae, and its polymeric chains are composed of glucuronate (G) and mannuronate (M) units in different proportions and motif blocks. Alginate hydrogels are easily obtained by crosslinking with divalent cations (e.g., $Ca^{2+}$), following an "egg-box" model where $Ca^{2+}$ ions are entrapped in cavities formed by the coupling between adjacent G units.

Some publications and patents have also explored the combination of alginate hydrogels with inorganic particles (i.e., hydroxyapatite or calcium phosphate, to promote osteogenesis; and silica to enhance the printability of the hydrogels), or particles obtained from synthetic polymers (i.e. poly(lactic acid), poly(glycolic acid) and polycaprolactone for the delivery of morphogenic growth factors).

The automated manufacturing is a process for the creation of three-dimensional objects from a digital model, for example in computer aided design (CAD), which is converted into standard triangulation (stl) language and subsequently divided in layers by slicing algorithm, thus creating detailed information about each transverse slice.

The creation of a printed component is carried out by means of additive manufacturing processes, wherein the object is created by the deposition of successive layers of material until the product is complete, where the successive layers of raw material can be in the form of hydrogels, pastes or powders. Each one of these layers can be seen as a horizontal transverse section in thin slices.

## TECHNICAL PROBLEMS

The interest in using alginate hydrogels for biomedical applications is driven by the interesting features of this polysaccharide, including the simple crosslinking and notable biocompatibility and biodegradability. However, alginate does not possess any cell binding moieties, which often results in low cell adhesion and reduced proliferation in the hydrogels, as it is disclosed in Neves, M. I., Moroni, L. & Barrias, C. C. Modulating Alginate Hydrogels for Improved Biological Performance as Cellular 3D Microenvironments. Front. Bioeng. Biotechnol. 8, (2020). Moreover, alginate-based hydrogels often underperform in terms of rheological and mechanical properties, that are fundamental for 3D printing processes, and show relatively unpredictable degradation rates. These limitations may hamper the long-term stability of the resulting 3D printed constructs, according to Kong, H. J., Kaigler, D., Kim, K. & Mooney, D. J. Controlling Rigidity and Degradation of Alginate Hydrogels via Molecular Weight Distribution. Biomacromolecules 5, 1720-1727 (2004); and Pahlevanzadeh, F. et al. Recent Trends in Three-Dimensional Bioinks Based on Alginate for Biomedical Applications. Materials (Basel). 13, 3980 (2020).

To circumvent these limitations, alginate is commonly combined with other materials, including other biopolymers like cellulose, chitosan, or collagen, to produce novel inks with improved properties. In particular, the mechanical reinforcement of alginate hydrogels with cellulose nanoforms (e.g., cellulose nanofibers, according to Han, C. et al. Effects of nanocellulose on alginate/gelatin bio-inks for extrusion-based 3D printing. BioResources 15, 7357-7373 (2020); and Nguyen, D. et al. Cartilage Tissue Engineering by the 3D Bioprinting of iPS Cells in a Nanocellulose/Alginate Bioink. Sci. Rep. 7, 658 (2017), bacterial cellulose, according to Wu, Z. et al. Biocompatibility evaluation of a 3D-bioprinted alginate-GelMA-bacterial nanocellulose (BNC) scaffold laden with oriented-growth RSC96 cells. Mater. Sci. Eng. C 129, 112393 (2021); and cellulose nanocrystals according to Wu, Y., Lin, Z. Y. (William), Wenger, A. C., Tam, K. C. & Tang, X. (Shirley). 3D bioprinting of liver-mimetic construct with alginate/cellulose nanocrystal hybrid bioink. Bioprinting 9, 1-6 (2018); leading to nanocomposite alginate hydrogel-based inks, has been reported in several studies, as appraised in Wang, X., Wang, Q. & Xu, C. Nanocellulose-based inks for 3D bioprinting: Key aspects in research development and challenging perspectives in applications - a mini review. Bioengineering 7, 40 (2020). Cellulose, the most abundant biopolymer in nature, can be obtained from plants, tunicates, algae, and some non-pathogenic bacteria, and it has notable mechanical properties. The inks obtained from alginate and cellulose reveal good biocompatibility, given the established non-toxic nature of both biopolymers, while showing enhanced mechanical performance, as disclosed in Hospodiuk, M., Dey, M., Sosnoski, D. & Ozbolat, I. T. The bioink: A comprehensive review on bioprintable materials. Biotechnol. Adv. 35, 217-239 (2017).

This approach has also been reported in several documents, for example the patent document WO2019173637A1, mentioned above, that describes a composite hydrogel containing nanocelluloses (nanofibers and nanocrystals) to reinforce alginate hydrogels for 3D bioprinting of cartilage. A similar approach is explored in the patent document WO2020245331A1, Berglund Linn and Oksman Kristiina, published on December 10th, 2020, using alginate and cellulose nanofibers obtained from brown seaweed. Additionally, the patent document WO2017210663A1, Gatenholm Paul, published on December 17th, 2017, discloses the use of nanocellulose and a cell-adhesion peptide (RGD)-conjugated alginate for improved 3D bioprinting of human skin (dermis).

In all these studies and documents, cellulose nanoforms are specifically used to reinforce the alginate hydrogels for application in extrusion 3D printing. The incorporation of bioactive compounds into these nanocellulose forms, prior to their incorporation into the hydrogels, could impart them with additional functionalities. However, the loading of these nanostructures, particularly with molecules with low water solubility, is still a challenge.

## SOLUTION TO PROBLEM

The present invention solves some of the problems of the state of the art by using spherical particles of cellulose or cellulose derivatives (such as cellulose acetate) for simultaneous reinforcement of alginate hydrogels and to impart additional functionalities. In the preferred embodiments, bioactive compounds are loaded in the particles, to promote the delivery of bioactive compounds from the 3D printed constructs. Additionally, if the inks are loaded with living cells before the printing process, these inks may even show interesting potential for 3D bioprinting of living tissues, due to the known biocompatible nature of these biopolymers.

## ADVANTAGEOUS EFFECTS OF INVENTION

The present invention constitutes a new strategy to circumvent some of the current limitations of alginate hydrogel inks for 3D printing applications, as the rheological and mechanical properties, while simultaneously imparting 3D printed constructs with new functionalities (drug releasing ability) and improved biological properties.

The cellulose or cellulose derivative-based particles are used to improve the rheological properties of the composition for additive manufacturing comprising a composite hydrogel, including the shear viscosity and the shear rate of said composition, while simultaneously improving the mechanical performance of the final 3D constructs, particularly their compression properties. The incorporation of cellulose or cellulose derivative-based particles into the alginate hydrogels improves their rheological properties for extrusion 3D printing, and the obtained printed constructs display better mechanical performance.

Additionally, the cellulose or cellulose derivative-based particles may be loaded with drugs or bioactive compounds prior to the printing process following different approaches, as the addition of the compounds to the cellulose solution prior to the regeneration step, allowing the final construct to possess drug-releasing potential, or to have improved biological properties and functionalities.

## BRIEF DESCRIPTION OF DRAWINGS

With the purpose of providing an understanding of the principles according to the embodiments of the present invention, reference will be made to the embodiments illustrated in the figures and to the terminology used to describe them. In any case, it must be understood that there is no intention of limiting the scope of the present invention to the content of the figures. Any subsequent alterations or modifications of the inventive characteristics shown herein, as well as any additional applications of the principles and embodiments of the invention shown, which would occur normally to a person skilled in the art having this description in hands, are considered as being within the scope of the claimed invention.

Figure 1 - Scanning Electron Microscopy (SEM) micrographs of cellulose acetate particles (CAp) obtained via water-on-polymer procedure;

Figure 2 - SEM micrographs of curcumin-loaded cellulose acetate particles (CApCUR) obtained via water-on-polymer procedure; and A) Photograph of the suspension of CApCUR, where the typical yellow tone of curcumin is clearly visible;

Figure 3 - Fourier transform infrared-Attenuated total reflection (FTIR-ATR) spectra of cellulose acetate, curcumin (CUR), CAp and CApCUR;

Figure 4 - Results of cell viability (%) of human keratinocytes (HaCaT) cells after 24 hours of exposure to CAp (A) and CApCUR (B) at different concentrations;

Figure 5 - Rheological properties, namely shear viscosity and shear stress, of ALG:CAp inks without the pre-crosslinking step.;

Figure 6 - Rheological properties, namely shear viscosity and shear stress, of ALG:CAp inks with a pre-crosslinking step (using 2 mL of a 0.5% (m/V) aqueous solution of $CaCl_2$ for a final total volume of 10 mL of ink);

Figure 7 - Recovery rate (%) of ALG:CAp inks, and of a control ALG ink for comparison purposes;

Figure 8 - Results (G' and G'' moduli) of the oscillatory tests performed on fully crosslinked ALG and ALG:CAp hydrogels;

Figure 9 - Rheological properties, namely shear viscosity and shear stress, of ALG:CApCUR inks without the pre-crosslinking step;

Figure 10 - Rheological properties, namely shear viscosity and shear stress, of ALG:CApCUR inks with a pre-crosslinking step (using 2 mL of a 0.5% (m/V) aqueous solution of $CaCl_2$ for a final total volume of 10 mL of ink);

Figure 11 - Recovery rate (%) of ALG:CApCUR inks, and of a control ALG ink for comparison purposes;

Figure 12 - (A) Results (G' and G'' moduli) of the oscillatory tests performed on fully crosslinked ALG and ALG:CApCUR hydrogels; (B) Photographs of the fully crosslinked hydrogels with CUR-loaded particles showing the increasing yellow tone with increasing CApCUR concentrations;

Figure 13 - Compression test results: Young's Modulus (A) and Compressive Stress (B) of the fully crosslinked hydrogels obtained from ALG, ALG:CAp and ALG:CApCUR inks;

Figure 14 - 3D printed lines extruded using different printing conditions for the optimization of the 3D printing parameters, here exemplified for ALG:CApCUR 10% ink. Printing pressure was evaluated from 0.5 (50 kPa) to 2.0 bar (200 kPa), and printing speed was varied from 5.0 to 15.0 mm.s$^{-1}$;

Figure 15 - 3D printed grid-like structures ($2 \times 2$ cm$^2$) with varying number of layers (2, 4 and 6) obtained using ALG, ALG:CAp and ALG:CApCUR inks;

Figure 16 - SEM micrographs of a 3D printed grid-like structure ($2 \times 2$ cm$^2$) obtained using the ALG ink;

Figure 17 - SEM micrographs of a 3D printed grid-like structure ($2 \times 2$ cm$^2$) obtained using the ALG:CApCUR _10 ink;

Figure 18 - Cumulative release (%) of CUR from fully crosslinked hydrogels for 24 hours in phosphate buffer saline (PBS) at 37 °C under mild agitation. The lines are for visual guidance only.

## DESCRIPTION OF EMBODIMENTS

The present invention describes a new biopolymeric composite hydrogel-based ink, composed of sodium alginate and cellulose or cellulose derivative-based spherical particles, for extrusion 3D printing purposes. The composite hydrogel-based ink is designed to be applied as an ink for 3D printing via extrusion techniques, for the layer-by-layer creation of 3D structures for several uses, for instance pharmaceutical and biomedical applications. The composite hydrogel-based ink is identified as a composition for additive manufacturing comprising a composite hydrogel.

The present invention refers, in a first aspect, to a composition for additive manufacturing comprising a composite hydrogel which further comprises:

a salt of alginic acid, referred to as alginate, in a concentration from 1 to 6%, wherein the percentual corresponds to the mass of alginate in relation to the volume of said composition; and spherical cellulose or cellulose derivative-based particles in a concentration from 1 to 20%, wherein the percentual corresponds to the mass of said spherical cellulose or cellulose derivative-based particles in relation to the mass of said alginate; and a cation of a pre-crosslinking salt in a concentration from 0.05 to 0.25%, wherein the percentual corresponds to the mass of said cation in relation to the total volume of the composition;

wherein the cation of alginate is selected from at least one of the group consisting of an alkali metal or an alkaline-earth metal; and wherein the spherical cellulose or cellulose derivative-based particles have a diameter from 0.1 to 2.0 micrometres; and wherein the cation of a pre-crosslinking salt is selected from at least one of a divalent or a trivalent cation.

In the preferred embodiments according to the present invention, the spherical cellulose or cellulose derivative-based particles have a diameter from 0.25 to 1.00 micrometre.

In the preferred embodiments according to the present invention, the cation of alginate is selected from at least one of the group consisting of magnesium, calcium, sodium or potassium. More preferably, the cation of alginate is sodium.

In the preferred embodiments according to the present invention, the cation of a pre-crosslinking salt is selected from at least one of the group consisting of an alkaline-earth metals, for example calcium or magnesium; or zinc; or iron.

In the preferred embodiments according to the present invention, the composition for additive manufacturing comprising a composite hydrogel is configured to be extruded in an additive manufacturing process.

In the preferred embodiments according to the present invention, the composition for additive manufacturing further comprises at least one of the group consisting of an active pharmaceutical ingredient, a bioactive compound, or living cells. The active pharmaceutical ingredient is at least one selected from the group comprising non-steroidal anti-inflammatory drugs (NSAIDs), anti-cancer drugs, and wound healing drugs. The NSAIDs may be selected from one or more compounds, for example, ibuprofen, naproxen, ketoprofen, flurbiprofen, and diclofenac. The anti-cancer drugs may be selected from one or more compounds, for example, doxorubicin, paclitaxel and docetaxel. The wound healing drugs may be selected from one or more drugs, for example, dexpanthenol, hyaluronic acid, and coenzyme Q10. The bioactive compound is at least one selected from the group consisting of phenolic compounds, including but not limited to flavonoids, isoflavonoids, phenolic acids, cinnamic acids, lignans, coumarins and curcuminoids, including the salts, esters, glucosides, and stereoisomers thereof. More preferably, the curcuminoid is curcumin.

In the preferred embodiments according to the present invention, the composition for additive manufacturing comprises cellulose or cellulose derivatives-based particles selected from the group consisting of cellulose, cellulose acetate, methyl cellulose, hydroxypropyl cellulose (HPC), hydroxyethyl cellulose (HEC), or hydroxypropylmethyl cellulose (HPMC) particles, or their mixtures. More preferably, the cellulose acetate particles comprise a mixture of diacetate and triacetate cellulose.

The present invention refers, in a second aspect, to a process for obtaining an additive manufacturing composition, as defined in the first aspect of the invention, comprising the following steps:

i. Preparing of an alginate aqueous solution in a concentration from 1 to 6%, wherein the percentual corresponds to the mass of the alginate in relation to the total volume of solvent;

ii. Preparing of an aqueous suspension of spherical cellulose or cellulose derivative-based particles in a concentration from 1 to 20%, wherein the percentual corresponds to the mass of said spherical cellulose or cellulose derivative-based particles in relation to the mass of said alginate, and wherein the spherical cellulose or cellulose derivative-based particles have a diameter from 0.1 to 2.00 micrometres;

iii. Mixing the alginate aqueous solution, obtained in step i), with the aqueous suspension of spherical cellulose or cellulose derivative-based particles, obtained in step ii);

iv. Performing a pre-crosslinking stage of the mixture obtained in the previous step with an aqueous solution comprising cations of a pre-crosslinking salt, wherein the cation of the pre-crosslinking salt is selected from at least one of the group consisting of a divalent or a trivalent cation;

wherein the steps i) and ii) are carried out in any order.

In the preferred embodiments according to the present invention, the step iv) is executed from about 1 minute to about 5 minutes. In the preferred embodiments according to the present invention, the step iv) is executed at a temperature from about 15 °C to about 30 °C.

In the preferred embodiments according to the present invention, the concentration of the cation of a pre-crosslinking salt in the aqueous solution is in the range from 0.1 to 2%, wherein the percentual corresponds to the mass of said cation in relation to the total volume of said solvent.

In the preferred embodiments according to the present invention, the sodium alginate is obtained from brown algae.

In the preferred embodiments according to the present invention, the spherical particles of cellulose or cellulose derivatives are obtained from cellulose acetate via a dissolution step followed by a regeneration step, as it is disclosed in Wondraczek, H., Petzold-Welcke, K., Fardim, P. & Heinze, T. Nanoparticles from conventional cellulose esters: evaluation of preparation methods. Cellulose 20, 751-760 (2013), and in Carvalho, J. P. F., Silva, A. C. Q., Silvestre, A. J. D., Freire, C. S. R. & Vilela, C. Spherical Cellulose Micro and Nanoparticles: A Review of Recent Developments and Applications. Nanomaterials 11, 2744 (2021). Therefore, the particles may be loaded with drugs or bioactive compounds prior to the printing process following different approaches, as it is known by a person skilled in the art, as the addition of the compounds to the cellulose solution prior to the regeneration step, allowing the final construct to possess drug-releasing abilities, or to have improved biological properties and functionalities.

In the preferred embodiments of the process for obtaining an additive manufacturing composition, the active pharmaceutical ingredient or the bioactive compound are included in the spherical particles of cellulose or cellulose derivative-based prior to step iii). A bioactive compound or a drug may be loaded in the cellulose particles previously to their incorporation into the alginate hydrogel, in order to grant them with compound-releasing abilities or improved biological properties. These compounds may be included in the particles during their manufacturing process or by posterior adsorption.

As an example, the pre-crosslinking step is carried out with an aqueous solution of 0.5% (w/V) of $CaCl_2$.

The present invention refers, in a third aspect, to a process of additive manufacturing of an object comprising the following steps:

a) preparation of a composition for additive manufacturing comprising a composite hydrogel, as defined in the first aspect of the invention;

b) deposition by a system configured for the manufacture of objects by additive manufacturing of a plurality of lines of said composition for additive manufacturing comprising a composite hydrogel over a printing table to form an object;

wherein the step b) comprises an extrusion step of said composition for additive manufacturing through a nozzle comprised in a head of the system configured for the manufacture of objects by additive manufacturing.

In the preferred embodiments of the process of additive manufacturing of an object, the object is selected from the group consisting of a biomedical device or a living tissue. Preferably, the biomedical device is a drug delivery-system, such as a patch, an adhesive, or a bandage. A living tissue may be used as a tissue regeneration material, such as an artificial skin graft.

In the preferred embodiments of the process of additive manufacturing of an object, after the step b) is performed a full-crosslinking stage of an additive manufactured object, wherein said additive manufactured object is immersed into an aqueous solution comprising cations of a full-crosslinking salt, wherein the cation of the full-crosslinking salt is selected from at least one of the group consisting of a divalent or a trivalent cation. The full-crosslinking stage maintain the integrity of the final additive manufactured object.

In the preferred embodiments according to the present invention, the full-crosslinking stage is executed from about 5 minutes to about 30 minutes. In the preferred embodiments according to the present invention, the full-crosslinking stage is executed at a temperature from about 15 °C to about 30 °C.

In the preferred embodiments according to the present invention, the cation of a full-crosslinking salt is selected from at least one of the group consisting of an alkaline-earth metal, for example calcium or magnesium; or zinc; or iron.

In the preferred embodiments according to the present invention, the concentration of the cation of a full-crosslinking salt in the aqueous solution is in the range from 0.1 to 5%, wherein the percentual corresponds to the mass of said cation in relation to the total volume of said solvent.

Example: Preparation of an alginate hydrogel ink reinforced with cellulose acetate particles loaded with curcumin for extrusion 3D printing

MATERIALS AND METHODS

Cellulose acetate (composed a mixture of diacetate and triacetate) was acquired from FILTER-LAB (Barcelona, Spain), while sodium alginate from brown algae (viscosity 4 - 12 cP at 1% in $H_2O$ at 25 °C) was obtained from Sigma-Aldrich (Sintra, Portugal). Calcium chloride anhydrous (96%) was provided by Carlo Erba Reagents (Barcelona, Spain). Acetone ($\geq$99.5%) was supplied by Honeywell (Charlotte, US), and curcumin (95%) was obtained from Alfa Aesar (Kandel, Germany). Ultrapure water (Type 1, 18.2 M$\Omega$·cm at 25 °C) was obtained by a Simplicity® Water Purification System (Merck, Darmstadt, Germany).

Dulbecco's Modified Eagle's Medium (DMEM) was purchased from PAN-Biotech (Germany). 3-(4,5-dimethylthiazol-2-yl)-2,5-diphenyltetrazolium bromide (MTT, 98%), dimethyl sulfoxide (DMSO, $\geq$99.9%), and trypsin-EDTA solution 10x (0.5% trypsin, 0.2% EDTA) were purchased from Sigma-Aldrich. Fetal bovine serum (FBS), phosphate buffer solution (PBS, pH 7.4), L-glutamine, penicillin/streptomycin and fungizone were obtained from Gibco® (Life Technologies, Carlsbad, CA, USA). L-Glutamine solution 200 mM and Penicillin/Streptomycin solution were purchased from Grisp (Porto, Portugal). Other reagents were of laboratory grade.

PREPARATION OF THE BIOPOLYMERIC COMPOSITE HYDROGEL INKS

Preparation of cellulose acetate spherical particles

Cellulose acetate particles (CAp) were prepared by a water-on-polymer method, as disclosed in Wondraczek, H., Petzold-Welcke, K., Fardim, P. & Heinze, T. Nanoparticles from conventional cellulose esters: evaluation of preparation methods. Cellulose 20, 751-760 (2013). Specifically, 80 mg of cellulose acetate were dissolved in 20 mL of acetone, under magnetic stirring at 500 rpm. A volume of 15 mL of ultrapure water was then added at a flow rate of 0.4 mL min$^{-1}$ using a Harvard Apparatus PHD Ultra syringe pump (Holliston, Massachusetts, EUA), equipped with a syringe with a 0.45 mm needle gauge, under continuous stirring at 500 rpm. The resulting suspension of cellulose acetate particles was washed twice with ultrapure water and stored in the refrigerator.

Preparation of curcumin-loaded cellulose acetate particles

Curcumin (CUR) loaded particles (CApCUR) were prepared using a similar method as described for the cellulose acetate particles. However, the process was performed in the dark given the photodegradability of CUR. Given so, 80 mg of cellulose acetate were dissolved in 20 mL of acetone containing 4 mg of CUR, under magnetic stirring at 500 rpm. 15 mL of distilled water were added at 0.4 mL/min, using a 0.45 mm needle, at 500 rpm. The resulting suspension of CApCUR was also washed with water twice, protected from light and stored in the refrigerator.

The incorporation of CUR (%) in the cellulose acetate particles was evaluated by ultraviolet-visible (UV-Vis) spectroscopy (Perkin-Elmer FT-IR System Spectrum BX spectrophotometer) based on the determination of the remaining CUR in solution, after centrifugation of CApCUR for 30 minutes at 6000 rpm. Absorbance was measured at 430 nm, and the concentration of CUR was calculated using a calibration curve (y = 0.0687x + 0.0181, r2 = 0.9995).

Preparation of the composite hydrogel inks

In a preferable embodiment of this invention, the alginate-based inks contain 4% (w/V) of alginate, and distinct amounts of CAp or CApCUR, namely 1%, 5% and 10% (wt.%) relative to the mass of ALG (Table 1). To achieve this, 400 mg of ALG and varying amounts of particles were mixed in 8 mL of ultrapure water, using moderate stirring overnight. These formulations were then pre-crosslinked by adding and 2 mL of a 0.5% (w/V) CaCl2 solution. The obtained inks were left to stabilize overnight at 4 °C.

Table 1 - Composition of the different compositions for additive manufacturing comprising a composite hydrogel according to the present invention

| Sample | ALG (mg) | CAp[1] % (wt.%) | mg | CApCUR[1] % (wt.%) | mg | Water mL | CaCl$_2$ 0.5%[2] mL |
|---|---|---|---|---|---|---|---|
| ALG:CAp_1 | 400 | 1 | 4 | - | | 8 | 2 |
| ALG:CAp_5 | 400 | 5 | 20 | - | | 8 | 2 |
| ALG:CAp_10 | 400 | 10 | 40 | - | | 8 | 2 |
| ALG:CApCUR _1 | 400 | - | | 1 | 4 | 8 | 2 |
| ALG:CApCUR _5 | 400 | - | | 5 | 20 | 8 | 2 |
| ALG:CApCUR _10 | 400 | - | | 10 | 40 | 8 | 2 |
| 1 CAp and CApCUR: % relative to the mass (mg) of ALG used; 2 CaCl$_2$ 0.5% on a w/V basis relative to the volume of CaCl$_2$ solution. | | | | | | | |

Characterization techniques

**Fourier transform infrared-Attenuated total reflection- (FTIR-ATR) spectroscopy**

FTIR-ATR spectra of the cellulose acetate, CAp, CUR and CApCUR samples were obtained using a Perkin-Elmer FT-IR System Spectrum BX spectrophotometer (Perkin-Elmer Inc., Waltham, MA, USA) equipped with a single horizontal Golden Gate ATR cell (Specac®, London, UK), at a range of 600-4000 cm$^{-1}$ and a resolution of 4 cm$^{-1}$ over 32 scans.

**Scanning electron microscopy (SEM)**

SEM micrographs of the CAp and CApCUR samples and of the 3D printed constructs were obtained using a HR-FESEM SU-70 Hitachi microscope (Hitachi High-Technologies Corporation, Tokyo, Japan) operating at 4 kV. The particle size determination was performed using ImageJ software to analyze at least 300 different particles on the SEM micrographs.

***In vitro* cytotoxicity assays of the cellulose acetate particles**

The cytotoxicity of CAp and CApCUR was assessed in human keratinocytes (HaCaT cell line) using the MTT assay, as it is disclosed in Mosmann, T. Rapid colorimetric assay for cellular growth and survival: application to proliferation and cytotoxicity assays. J. Immunol. Methods 65, 55-63 (1983). To achieve this, cells were cultivated in complete DMEM supplemented with 10% FBS, 2 mM L-glutamine, 10000 U mL$^{-1}$ penicillin/streptomycin and 250 $\mu$g mL$^{-1}$ fungizone at 37 °C in a 5% $CO_2$ humidified atmosphere. Cells were observed daily using an inverted phase-contrast Eclipse TS100 microscope (Nikon, Tokyo, Japan). All the essays were performed in triplicate.

Suspensions of particles were sterilized by ultraviolet irradiation, and then incubated in DMEM at 37 °C with 5% $CO_2$ for 24 hours. Different amounts of particles were used to obtain analogue concentrations to the seen in the inks' formulations. As negative controls, HaCaT cells were treated identically as described for the samples but exposed only to DMEM.

In the meantime, one 96-well plate was prepared with 5×4 wells filled with 6000 cells/well, and then incubated for 24 hours for adhesion. After that time, the cell culture medium in the wells was replaced by 100 $\mu$L of each sample, and cells were further incubated for 24 hours.

At the end of the incubation time, a 50 $\mu$L aliquot of MTT (1 g L$^{-1}$) was added to each well and incubated for 4 hours at 37 °C in a 5% $CO_2$ humidified atmosphere. After that, the culture medium was replaced with 150 $\mu$L of DMSO and the plate was placed in an orbital shaker for 2 hours in the dark. The absorbance of the samples was measured with a BioTek Synergy HT plate reader (Synergy HT Multi-Mode, BioTeK, Winooski, VT) at 570 nm with blank corrections. The cell viability was calculated with respect to the controls by the formula:

$$\text{Cell viability (\%)} = \left[\left(Abs_{sample} - Abs_{DMSO}\right)/\left(Abs_{control} - Abs_{DMSO}\right)\right] \times 100$$

where Abs$_{sample}$ is the absorbance of the samples, Abs$_{DMSO}$ is the absorbance of DMSO and Abs$_{control}$ is the absorbance of the control.

**Rheological analysis of the inks and the fully cross-linked hydrogels**

The rheological properties of the inks and fully crosslinked hydrogels were evaluated by rotational and oscillatory tests, performed using a Kinexus Pro Rheometer (Malvern Instruments Limited, Malvern, United Kingdom) with a cone-plate geometry (cone angle of 4° and diameter of 40 mm). Rotational tests were performed on the hydrogel inks using a measurement gap of 1 mm and a shear rate of $0.1\text{-}100 \text{ s}^{-1}$. The oscillatory tests used to determine the storage (G') and loss (G") moduli of the fully crosslinked hydrogels were performed at 1 Hz frequency and a shear strain of 0-100%, using cylinder-shaped (10 mm diameter, 5 mm height) samples of the hydrogels crosslinked overnight in a 2% (m/V) $CaCl_2$ solution.

The recovery rate of the inks was evaluated using a 3-step oscillatory test: (i) measurement of G' in relaxation, at 1 Pa for 1 min; (ii) measurement of the G' under stress, at 100 Pa for 10 s; and (iii) a second measurement of the G' in a relaxation stage, at 1 Pa for 1 min. The recovery (%) was calculated according to the equation:

$$Recovery\ (\%)\ = \frac{G'_{Recovered}}{G'_{Initial}} \times 100$$

where $G'_{Initial}$ is the average storage modulus in the initial relaxation phase, and $G'_{Recovered}$ corresponds to the storage modulus measured 5 s after the shear stress reduced from 100 Pa back to 1 Pa.

All the rheological measurements were performed at 20 °C, using a water lock to prevent dehydration of the samples and a Peltier module for temperature control.

**Mechanical compression tests**

Mechanical compression tests were performed on at least five similar cylindrical samples of fully crosslinked hydrogels obtained from the ALG, ALG:CAp and ALG:CApCUR inks. The samples, with a diameter of 10 mm and height of 5 mm, were tested using a uniaxial Instron 5966 machine (Instron Corporation, USA) in the compression mode, using a static load cell of 500 N at $5 \text{ mm.min}^{-1}$. All tests were performed up to 80% of strain, and the results obtained were calculated using the Bluehill 3 Software.

**3D printing assays**

All printing essays were performed on a 3D-Bioplotter from EnvisionTEC (Germany). The printing parameters (pressure and printing speed) were first optimized by printing straight filaments with around 10 cm length using a 0.25 mm nozzle (inner diameter) at different speeds ($5\text{-}15 \text{ mm.s}^{-1}$) and air pressures (0.5 bar (50 kPa) to 2.0 bar (200kPa)).

Then, the design of the 3D printed structures was previously developed using appropriate 3D-modelling software to model grid-like structures with dimensions of $20 \times 20 \text{ mm}^2$ and a spacing of 2.25 mm between filaments.

Structures with varying layers were 3D printed at 20 °C using the ALG, ALG:CAp and ALG:CApCUR inks, and fully crosslinked with an aqueous solution of 2% (m/V) of $CaCl_2$.

**Curcumin-release studies**

The release of the model bioactive compound (CUR) from the fully crosslinked hydrogels was evaluated by dipping them in 40 mL of PBS at 37 °C, under moderate agitation for 24 hours. To achieve this, hydrogels were obtained by using 3 mL of the ink formulation with higher curcumin content (ALG:CApCUR_10 ink). The essay was performed in triplicate. This ink was chosen given its higher content of CApCUR, which would result in higher CUR-release.

Aliquots of 2 mL were collected at selected time points, and the collected medium was replaced with fresh medium, preheated at 37 °C. The amount of CUR released into the media was measured by UV-Vis spectroscopy (using a Thermo Scientific Evolution UV-Vis 600, Thermo Fisher Scientific) at 430 nm. The cumulative release was calculated using the formula:

$$C_{cumulative} = C_n + [(2 \times C_{n-1})/40]$$

where $C_n$ and $C_{n-1}$ are the concentrations of CUR in solution at times *n* and *n-1.*

RESULTS AND DISCUSSION

**Characterization of the Cellulose Acetate Particles**

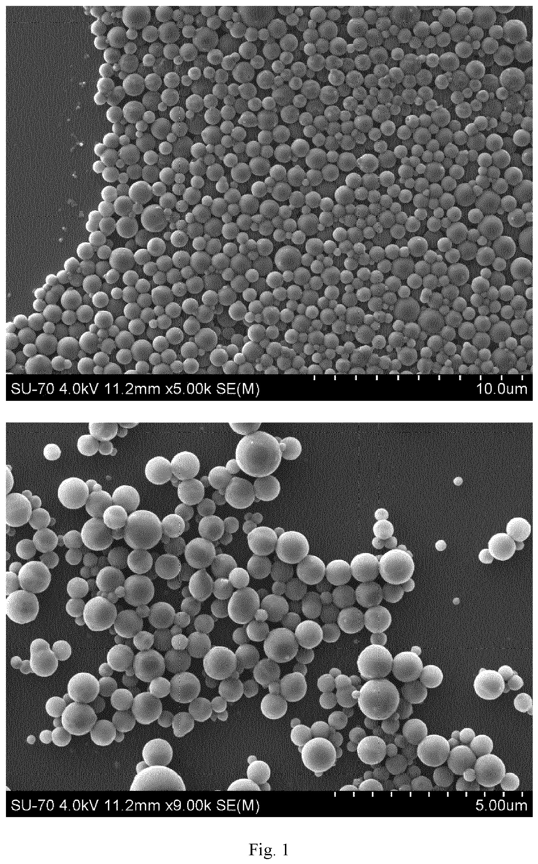

The SEM micrographs of the obtained cellulose acetate particles (CAp) confirm that the used water-on-polymer method originates spherical, individualized, and smooth particles **(Figure 1).** The size measurements (using ImageJ software) revealed that these spherical cellulose derivative-based particles display an average diameter of 717 $\pm$ 196 nm.

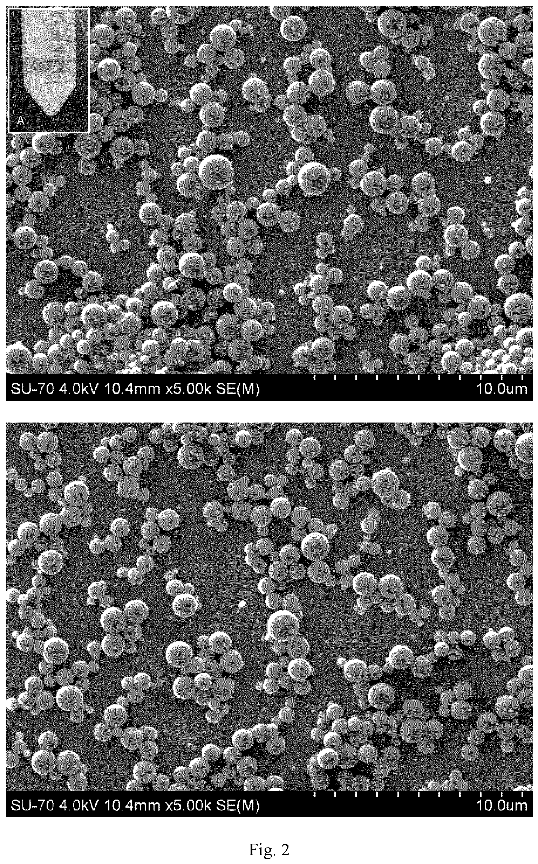

Moreover, as observed in the SEM micrographs **(Figure 2),** the particles produced using a cellulose acetate solution containing curcumin (CApCUR) have similar morphological features when compared to the cellulose acetate counterparts, with sizes of 749 $\pm$ 232 nm. The CApCUR particles are equally spherical, smooth, and well dispersed and individualized. In sum, the incorporation of CUR in the process has no significant impact on the morphology and size distribution of the cellulose acetate particles.

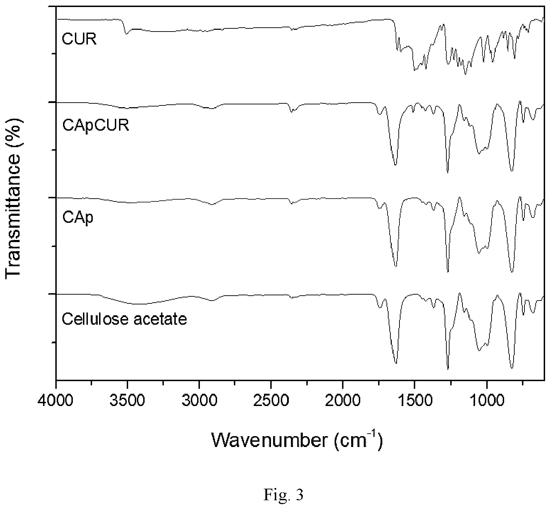

The FTIR-ATR spectroscopic analysis **(Figure 3)** of the raw materials (cellulose acetate and curcumin) and of the obtained particles (CAp and CApCUR) confirms that the production process and the incorporation of curcumin does not affect the structure of this cellulose derivative, given the close resemblance of the spectra of the particles with the one of pristine cellulose acetate, with main vibrations at 3416 cm$^{-1}$ (-OH stretching), 2918 cm$^{-1}$ (symmetric C-H stretching), 1633 cm$^{-1}$ (C=O stretching), 1371 cm$^{-1}$ (C-H bending of the $CH_3$ in the acetyl group) and 1275 cm$^{-1}$ (C-O stretching). In the spectrum of the CApCUR particles, the emergence of a peak at 1513 cm$^{-1}$, characteristic of CUR, is an indication of the successful incorporation of this compound in the cellulose acetate particles. Furthermore, the presence of curcumin in the cellulose acetate particles (CApCUR) is indeed confirmed by the vibrant yellow colour of the spheres under suspension **(Figure 2).** An incorporation rate of 81% relative to the initial mass of curcumin added was determined by UV-Vis spectroscopy analysis of the curcumin content in the supernatant solution obtained after the fabrication step. This incorporation is similar to the results obtained for the incorporation of CUR in particles from other materials, and corroborates the potential of cellulose acetate for the production of particles incorporating CUR.

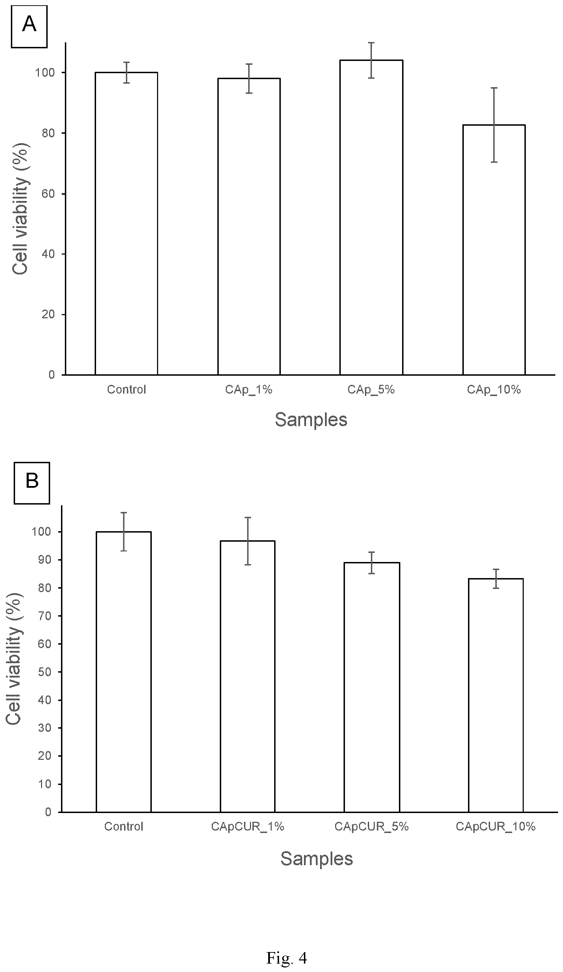

The determination of the cytotoxicity of the CAp and CApCUR was assessed against HaCaT cells using the MTT assay after 24 hours of exposure. This cell line was selected considering the potential applications of the developed inks for the 3D printing of constructs for topical/transdermal drug delivery or skin regeneration, for instance. As displayed in **Figure 4A,** the CAp particles showed no significant cytotoxic effect, with cell viabilities of 98.0 $\pm$ 3.4%, 104.8 $\pm$ 5.8% and 82.7 $\pm$ 12.3% for concentrations of 1, 5 and 10% particles, respectively, when compared with the control. Similarly, the CApCUR counterparts reveal no cytotoxic effect in the concentrations tested, as seen in **Figure 4B,** with 96.7 $\pm$ 8.4%, 88.9 $\pm$ 3.8% and 83.3 $\pm$ 3.4% viabilities after 24 hours for 1, 5 and 10% concentrations. All these cell viabilities are higher than the threshold of 70% cell viability, and therefore, the particles can be considered non-cytotoxic for HaCaT cells in these concentrations, as defined by the ISO 10993-5:2009 for the evaluation of the cytotoxicity of the materials.

**Characterization of the ALG:CAp hydrogel inks**

The rheologic properties have a very important role on the extrusion printability of hydrogel-based inks. Given so, the behaviour of the hydrogels under stress was studied, to understand how these materials would perform along the 3D printing process.

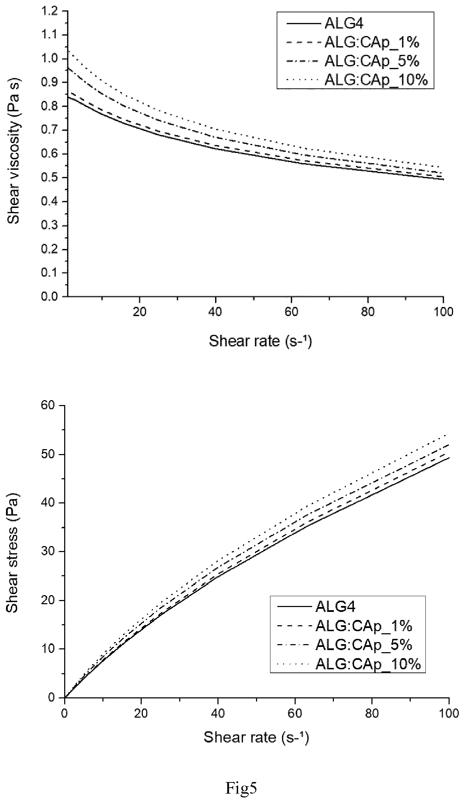

First, the impact of the pre-crosslinking step on the characteristics of these hydrogel inks was assessed. The viscosity and shear stress (as a function of shear rate) were initially evaluated for the mixtures of alginate and CAp without pre-crosslinking. The results shown in **Figure 5,** reveal a slight increase on these parameters with the increasing content of cellulose acetate particles, suggesting an improvement of the rheological properties with the addition of these cellulose derivative-based spherical particles. However, these rheological properties are still not ideal for extrusion printing. Yet, the results confirm that all inks show a shear-thinning behaviour, viz. a decrease of shear viscosity with the increase of the shear rates applied.

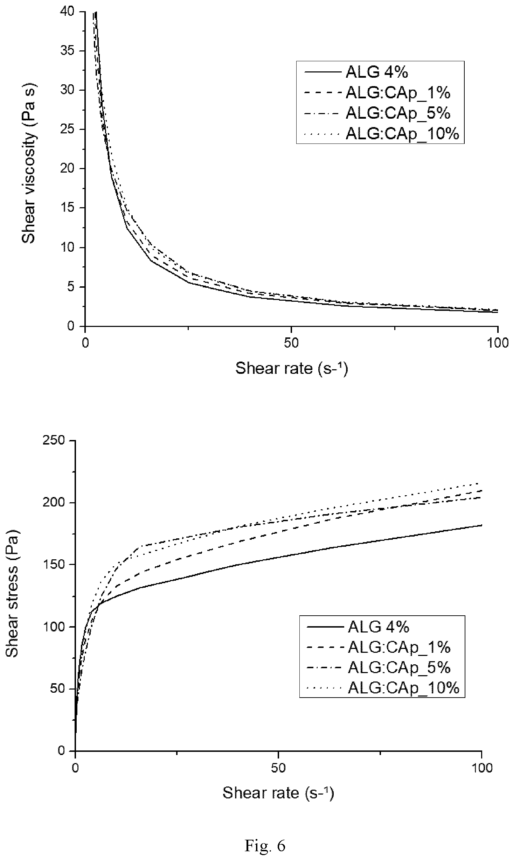

Pre-crosslinked hydrogel inks were then evaluated for the same parameters. As shown in **Figure 6,** the pre-crosslinked inks reveal higher shear viscosities and shear stress, given the pre-crosslinking of the hydrogels, and a very clear shear-thinning behaviour. Additionally, there seems to be an impact of the presence of CAp in these parameters, with an increase on both shear viscosity and shear stress in ALG:CAp inks, when compared with the single ALG counterpart.

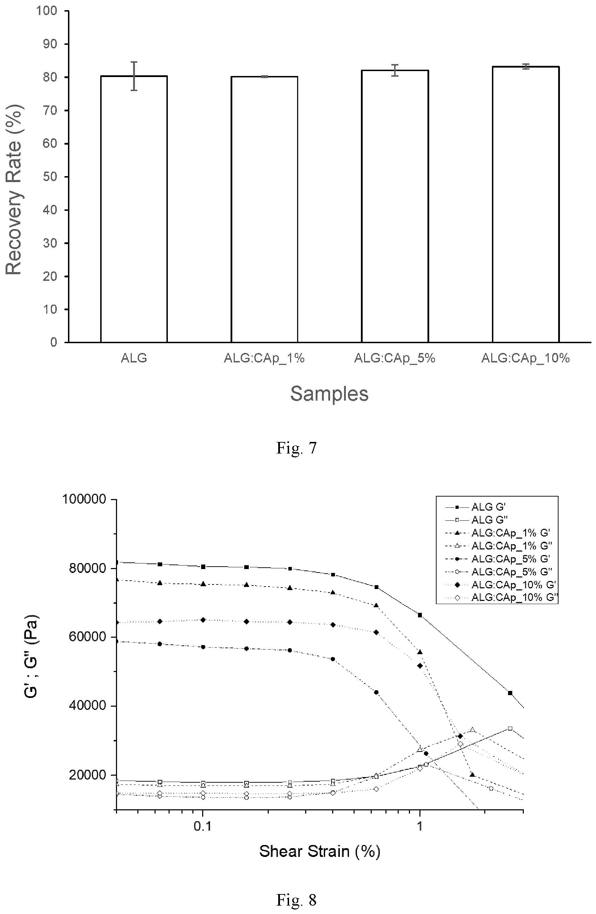

Afterwards, the recovery rate of the formulations was assessed in order to understand how the rheological properties of the inks is recovered after being subjected to the forces applied in the printing process. The data, summarized in **Figure 7,** shows that pristine alginate hydrogels show a recovery rate of 80.34 $\pm$ 4.28%, and that the composite inks containing cellulose acetate particles have recovery rates of 80.17 $\pm$ 0.20%, 82.09 $\pm$ 1.67% and 83.21 $\pm$ 0.73% for the ALG:CAp_1%, ALG:CAp_5% and ALG:CAp_10%, respectively. Therefore, all inks present very high recovery rates (above 80%), and the recovery rate does not seem to be affected by the presence and different contents of CAp for the investigated range. Considering all these data, the viscosity, shear rate and recovery rate of all of these inks seems adequate for extrusion 3D printing applications.

The subsequent evaluation of the G' and G'' moduli of fully crosslinked hydrogels obtained from these hydrogel inks was performed to enlighten their viscoelasticity. The values obtained for the elastic modulus are significantly higher than the viscous counterpart (G'>G'') for all samples **(Figure 8),** indicating that all fully crosslinked hydrogels have a solid-like behaviour, as desired for integer and robust 3D printed constructs.

## Characterization of the ALG:CApCUR inks

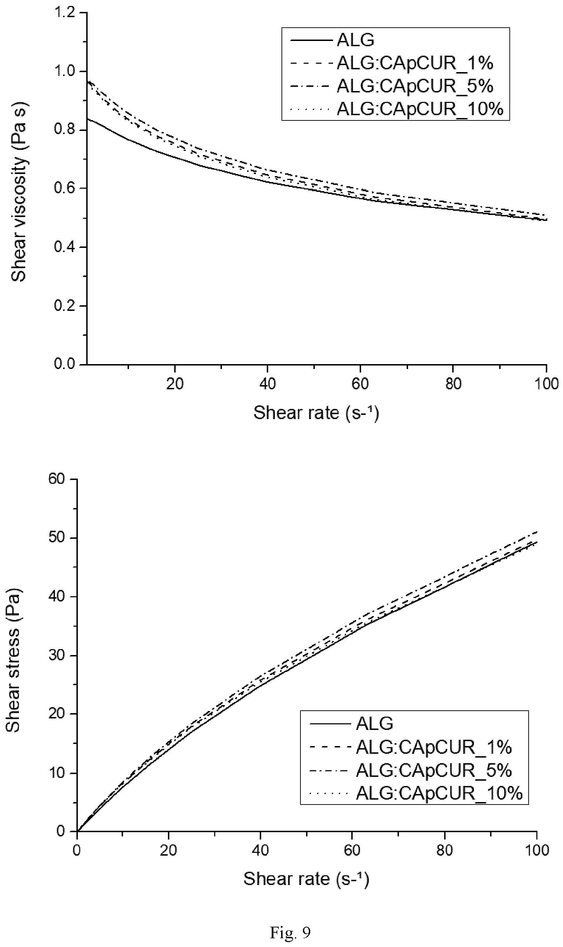

All the rheological properties assessed for the CAp particles were also evaluated for the CApCUR counterparts, aiming to understand how the incorporation of CUR could impact the characteristics of the inks. As may be seen in **Figure 9,** for the formulations without pre-crosslinking, the tendency observed above for the CAp inks was confirmed, with increasing amounts of CApCUR particles leading to higher shear viscosities and shear stress. The shear-thinning behaviour is also observable for these mixtures.

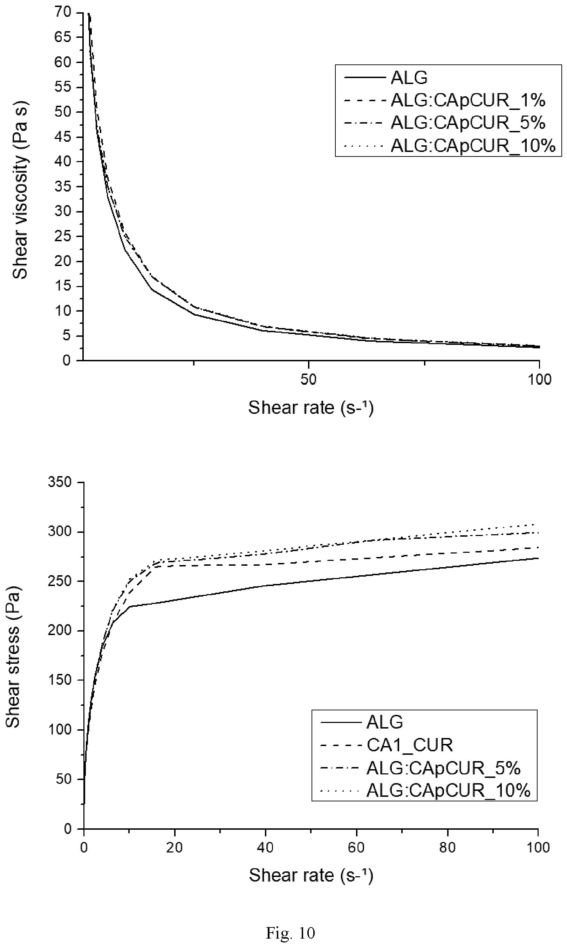

The effect of the pre-crosslinking step on the rheological characteristics of these mixtures is also very relevant **(Figure 10)** with the values of shear stress and shear viscosity at max shear rate increasing from around 50 Pa and 0.5 Pa.s to above 250 Pa and 4 Pa.s, respectively. Once more, the shear thinning behaviour is more evident from the pre-crosslinked hydrogels.

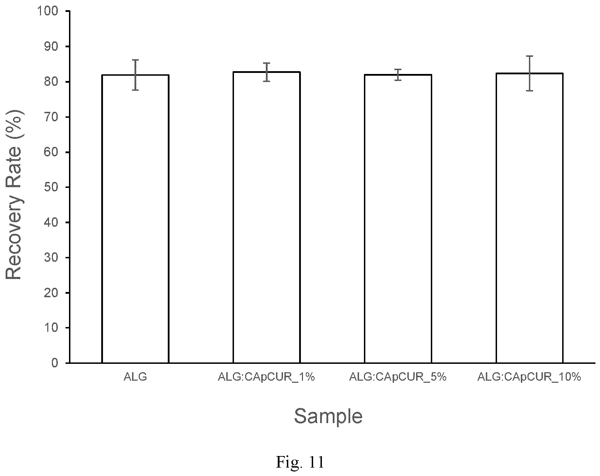

Additionally, the recovery rates are not affected by the incorporation of CApCUR in the hydrogels **(Figure 11),** with the ALG:CApCur_1% showing $81.92 \pm 4.23\%$, ALG:CApCUR_5% with $81.95 \pm 1.52\%$ and ALG:CApCUR_10% with $82.34 \pm 4.90\%$. Again, the recovery rates of these inks are higher than 80%, and very similar to those of the hydrogels obtained using cellulose acetate particles without curcumin.

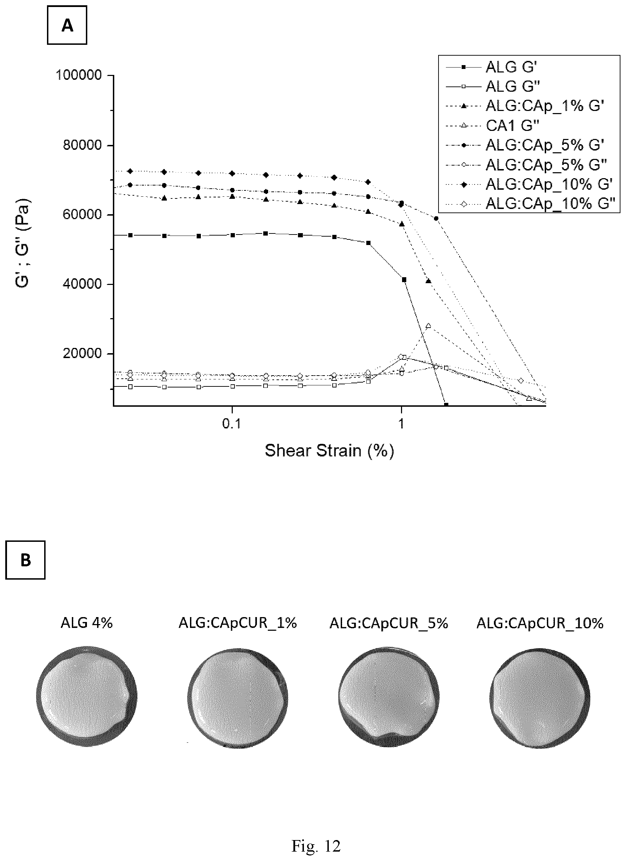

The viscoelastic properties of the corresponding fully crosslinked hydrogels **(Figure 12)** also show an analogous solid-like behaviour, with all ink samples displaying G' values above the G''. Interestingly, the incorporation of CUR-loaded CA particles in these hydrogels is easily noticed by the substantial change in their colour **(Figure 12A),** with the yellow colour increasing notoriously with the increasing concentrations of CApCUR.

Considering these data, the incorporation of curcumin in the cellulose acetate particles did not affect the rheological properties of the alginate hydrogel inks with or without pre-crosslinking, and therefore does not compromise the potential of the pre-crosslinked inks for 3D extrusion bioprinting.

## Mechanical compression tests of the ALG:CApCUR hydrogels

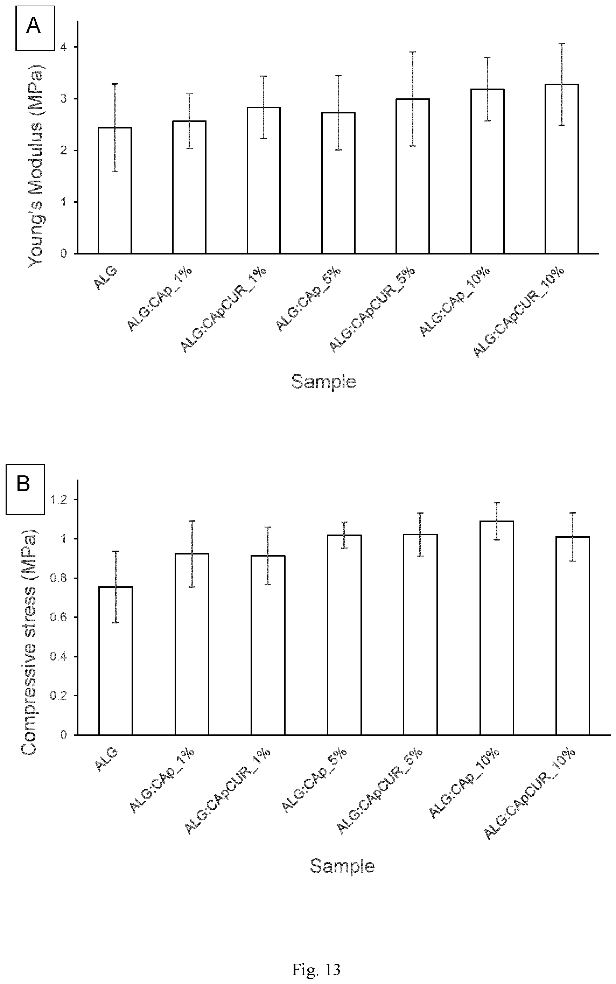

The mechanical properties of fully crosslinked hydrogels obtained from ALG:CAp and ALG:CApCUR inks were assessed using compression assays. To understand how the presence of both types of particles (CAp and CApCUR) would impact these properties, a hydrogel composed exclusively of ALG was also tested. The results of these assays, indicate that that the Young's Modulus **(Figure 13A)** of the hydrogels seems to increase with the content of particles. In fact, ALG hydrogels show values of $2.43 \pm 0.84$ MPa, while CAp containing hydrogels show values of $2.56 \pm 0.53$ MPa (CAp_1%), $2.73 \pm 0.71$ MPa (CAp_5%) and $3.18 \pm 0.61$ MPa (CAp_10%). A similar increase is seen for CApCUR composite hydrogels with $2.82 \pm 0.60$ MPa, $2.99 \pm 0.91$ MPa and $3.28 \pm 0.79$ MPa, for CApCUR concentrations of 1%, 5% and 10% respectively.

The evaluation of the compressive stress of these hydrogels at 80% strain also seems to show an increase with the presence (and increasing concentrations) of particles in the inks (Figure 13 B), with ALG showing the lowest value at $0.75 \pm 0.18$ MPa, and CAp / CApCUR samples revealing higher values and analogue results for the same concentrations. Specifically, CAp_1% and CApCUR_1% at $0.92 \pm 0.17$ MPa and $0.91 \pm 0.14$ MPa, respectively; CAp_5% and CApCUR_5% with $1.01 \pm 0.07$ MPa and $1.02 \pm 0.10$ MPa respectively; and finally, CAp_10% and CApCUR_10% with $1.08 \pm 0.09$ MPa and $1.01 \pm 0.12$ MPa.

These results confirm that the incorporation of CApCUR enhances the mechanical properties of the fully crosslinked alginate-based hydrogels.

## 3D printing assays

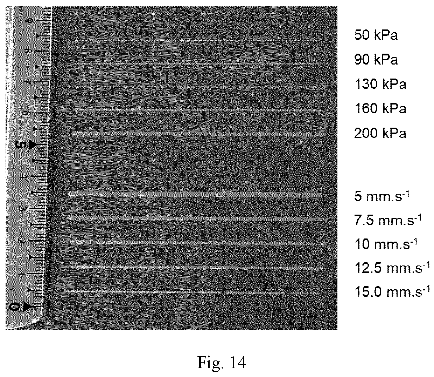

The optimization of the extrusion printing conditions was performed initially by printing straight filaments of the inks. The obtained results show that the inks with CAp and CApCUR could be successfully printed in the same conditions: at 20 °C, using a printing speed of 10 mm.s$^{-1}$ and a pressure of 2 bar (200 kPa), the process originated an integer and defined filament and dispensed moderate amounts of the inks, as illustrated for the CApCUR inks in **Figure 14**.

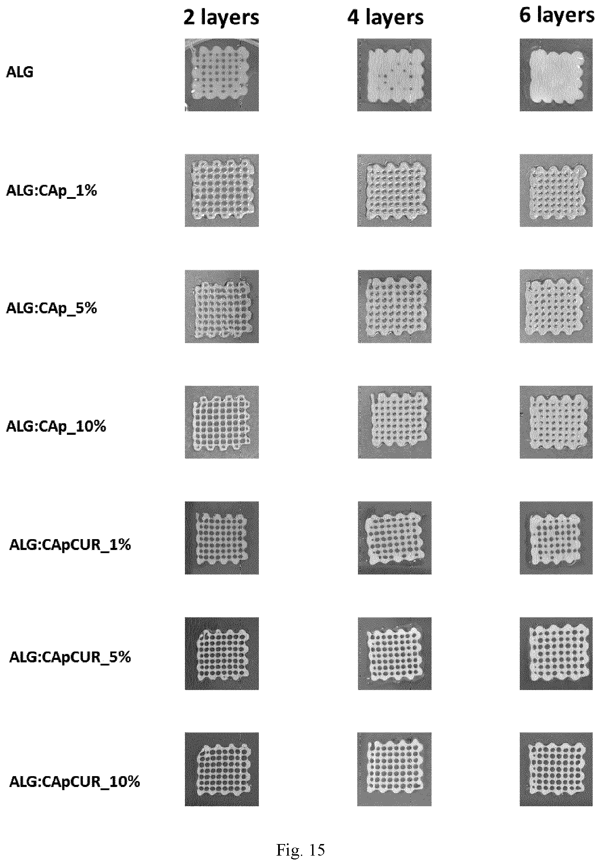

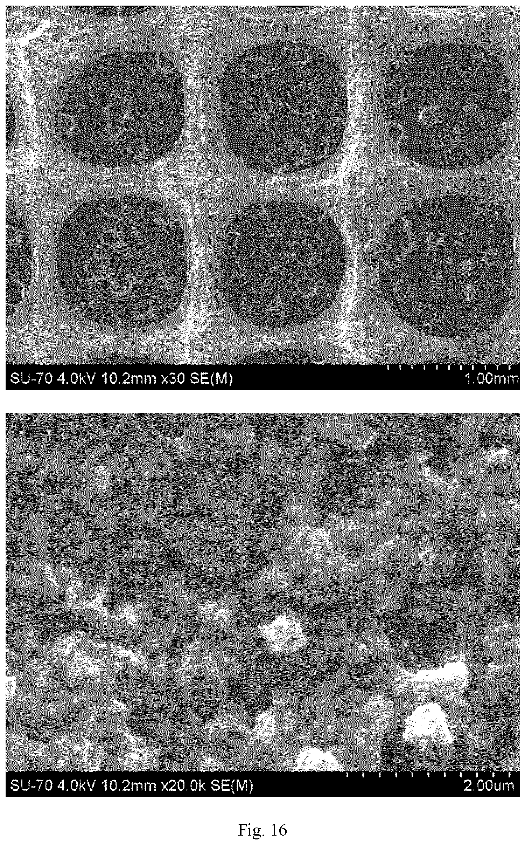

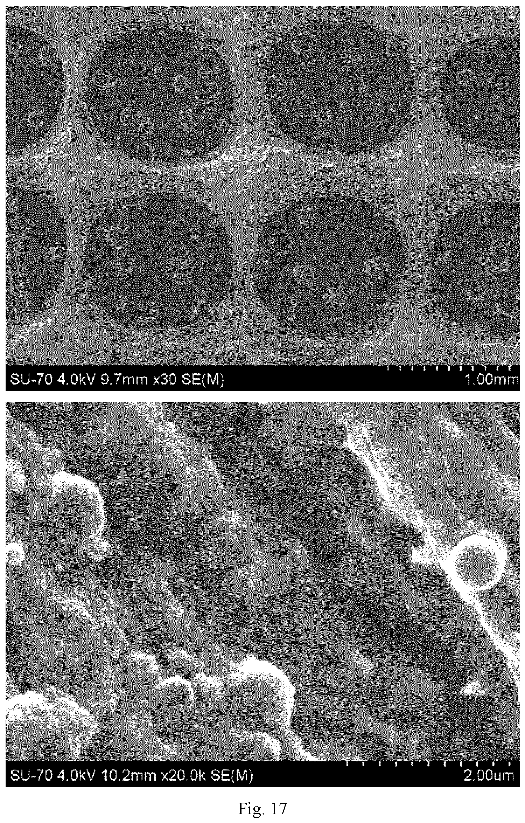

With this in mind, grid-like 3D structures with various numbers of layers were printed using ALG, ALG:CAp and ALG:CApCUR inks. The structures printed using ALG:CApCUR **(Figure 15)**, show the lively yellow colour of CUR, with higher concentration of particles leading to a more notorious yellow tone, when compared with structures obtained exclusively from ALG or ALG:CAp. Additionally, all the 3D printed constructs with the composite hydrogel inks (ALG:CAp and ALG_CApCUR) demonstrated better resolution, with a higher definition of the grid-like structure, when compared with the structures obtained from the ALG ink. Moreover, the SEM micrographs of the 3D structures obtained from ALG (**Figure 16**) and ALG:CApCUR_10% **(Figure 17)** show that the structures obtained with the composite hydrogels possess spherical particles on the surface and embed in the matrix (which are absent in the ALG counterpart), confirming the presence of the CUR-loaded cellulose acetate particles in these structures, even after the extrusion 3D printing process.

Therefore, the use of ALG:CApCUR and ALG:CApCUR reinforced inks improves the outcome of the 3D printing procedure, and these inks may be used for the enhanced printing of detailed multi-layered 3D structures.

### Release of CUR from 3D printed structures

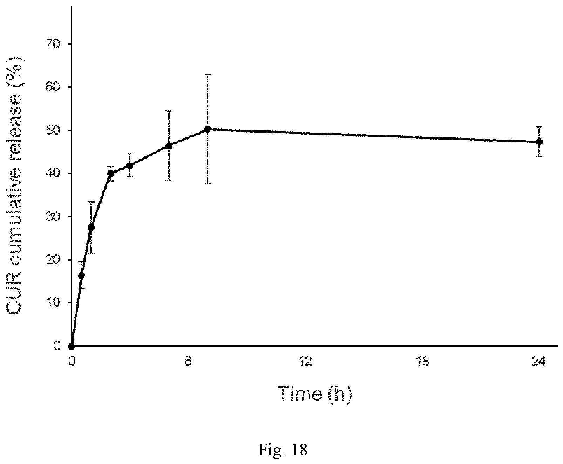

The release of curcumin from the fully crosslinked hydrogels was evaluated for a period of 24 hours through their emersion in PBS, at 37 °C. As depicted in **Figure 18,** after an initial burst, the cumulative release of CUR reaches a plateau after around 7 hours, achieving a final cumulative release of nearly 50%.

These data confirm the possibility of using CApCUR to provide ALG hydrogels with the capability of releasing bioactive compounds with low water solubility to the media.

As used in this description, the expressions "composite hydrogel ink" or "composite hydrogel-based ink" refer to a "composition for additive manufacturing comprising a composite hydrogel".

As used in this description, the expression "3D printing" refers to a "process of additive manufacturing".

As used in this description, the pressure values refer to values above the atmospheric pressure.

As used in this description, the expressions "around" and "approximately" refer to an interval of values of more or less 10% of the number specified.

Moreover, the term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A; X employs B; or X employs both A and B.

In addition, the articles "a" and "an" as used in this application and the appended claims should generally be construed to mean "one or more" unless specified otherwise or clear from the context to be directed to a singular form.

Further, as used herein, the term "exemplary" is intended to mean serving as an illustration or example of something and is not intended to indicate a preference.

As used in this description, the expression "substantially" means that the real value is within the interval of around 10% of the desired value, variable or related limit, particularly within around 5% of the desired value, variable or related limit or specially within the 1% of the desired value, variable or related limit.

As used in this description, the spherical cellulose or cellulose derivative-based particles comprise substantially 100% of the polymer.

The subject matter described above is provided as an illustration of the present invention and, therefore, cannot be interpreted so as to limit it. The terminology used herein with the purpose of describing preferred embodiments of the present invention, must not be interpreted to limit the invention. As used in the specification, the definite and indefinite articles, in their singular form, aim at the interpretation of also including the plural forms, unless the context of the description indicates, explicitly, the contrary. It will be understood that the expressions "comprise" and "include", when used in this description, specify the presence of the characteristics, the elements, the components, the steps and the related operations, however, they do not exclude the possibility of other characteristics, elements, components, steps and operations also being contemplated.

### LIST OF CITATIONS

Follows the list of citations:

### PATENT LITERATURE

WO2021234141A1, Guasch Camell Judit et al, published on November 25th, 2021;
WO2019173637A1, Nelson Kimberly et al, published on September 12th, 2019;
WO2020081982A1, Ogle Brenda M et al, published on April 23rd, 2020;
WO2020245331A1, Berglund Linn and Oksman Kristiina, published on December 10th, 2020;
WO2017210663A1, Gatenholm Paul, published on December 17th, 2017.

**NON-PATENT LITERATURE**

Schwab, A. et al. Printability and Shape Fidelity of Bioinks in 3D Bioprinting. Chem. Rev. 120, 11028-11055 (2020);
Unagolla, J. M. & Jayasuriya, A. C. Hydrogel-based 3D bioprinting: A comprehensive review on cell-laden hydrogels, bioink formulations, and future perspectives. Appl. Mater. Today 18, 100479 (2020);
Kumar, S., Tharayil, A. & Thomas, S. 3D Bioprinting of Nature-Inspired Hydrogel Inks Based on Synthetic Polymers. ACS Appl. Polym. Mater. 3, 3685-3701 (2021);
Datta, S., Barua, R. & Das, J. Importance of Alginate Bioink for 3D Bioprinting in Tissue Engineering and Regenerative Medicine. in Alginates - Recent Uses of This Natural Polymer (IntechOpen, 2020). doi:10.5772/intechopen.90426;
Taghizadeh, M. et al. Chitosan-based inks for 3D printing and bioprinting. Green Chem. 24, 62-101 (2022);
Wang, X. et al. Gelatin-Based Hydrogels for Organ 3D Bioprinting. Polymers (Basel). 9, 401 (2017);
Neves, M. I., Moroni, L. & Barrias, C. C. Modulating Alginate Hydrogels for Improved Biological Performance as Cellular 3D Microenvironments. Front. Bioeng. Biotechnol. 8, (2020);
Kong, H. J., Kaigler, D., Kim, K. & Mooney, D. J. Controlling Rigidity and Degradation of Alginate Hydrogels via Molecular Weight Distribution. Biomacromolecules 5, 1720-1727 (2004);
Pahlevanzadeh, F. et al. Recent Trends in Three-Dimensional Bioinks Based on Alginate for Biomedical Applications. Materials (Basel). 13, 3980 (2020);
Han, C. et al. Effects of nanocellulose on alginate/gelatin bio-inks for extrusion-based 3D printing. BioResources 15, 7357-7373 (2020);
Nguyen, D. et al. Cartilage Tissue Engineering by the 3D Bioprinting of iPS Cells in a Nanocellulose/Alginate Bioink. Sci. Rep. 7, 658 (2017);
Wu, Z. et al. Biocompatibility evaluation of a 3D-bioprinted alginate-GelMA-bacteria nanocellulose (BNC) scaffold laden with oriented-growth RSC96 cells. Mater. Sci. Eng. C 129, 112393 (2021);
Wu, Y., Lin, Z. Y. (William), Wenger, A. C., Tam, K. C. & Tang, X. (Shirley). 3D bioprinting of liver-mimetic construct with alginate/cellulose nanocrystal hybrid bioink. Bioprinting 9, 1-6 (2018);
Wang, X., Wang, Q. & Xu, C. Nanocellulose-based inks for 3d bioprinting: Key aspects in research development and challenging perspectives in applications-a mini review. Bioengineering 7, 40 (2020);
Hospodiuk, M., Dey, M., Sosnoski, D. & Ozbolat, I. T. The bioink: A comprehensive review on bioprintable materials. Biotechnol. Adv. 35, 217-239 (2017);
Wondraczek, H., Petzold-Welcke, K., Fardim, P. & Heinze, T. Nanoparticles from conventional cellulose esters: evaluation of preparation methods. Cellulose 20, 751-760 (2013);
Carvalho, J. P. F., Silva, A. C. Q., Silvestre, A. J. D., Freire, C. S. R. & Vilela, C. Spherical Cellulose Micro and Nanoparticles: A Review of Recent Developments and Applications. Nanomaterials 11, 2744 (2021);
Mosmann, T. Rapid colorimetric assay for cellular growth and survival: application to proliferation and cytotoxicity assays. J. Immunol. Methods 65, 55-63 (1983);

**Claims**

1. A composition for additive manufacturing comprising a composite hydrogel **characterized by** comprising:

   a salt of alginic acid, referred to as alginate, in a concentration from 1 to 6%, wherein the percentual corresponds to the mass of alginate in relation to the volume of said composition; and
   spherical cellulose or cellulose derivative-based particles in a concentration from 1 to 20%, wherein the percentual corresponds to the mass of said spherical cellulose or cellulose derivative-based particles in relation to the mass of said alginate; and
   a cation of a pre-crosslinking salt in a concentration from 0.05 to 0.25%, wherein the percentual corresponds to the mass of said cation in relation to the total volume of the composition;
   wherein the cation of alginate is selected from at least one of the group consisting of an alkali metal or an alkaline-earth metal; and
   wherein the spherical cellulose or cellulose derivative-based particles have a diameter from 0.1 to 2.0 micrometres; and
   wherein the cation of a pre-crosslinking salt is selected from at least one of a divalent or a trivalent cation.

2. The composition for additive manufacturing comprising a composite hydrogel, according to the previous claim, **characterized in that** said composition is configured to be extruded in an additive manufacturing process.

3. The composition for additive manufacturing comprising a composite hydrogel, according to any one of the previous claims, **characterized by** further comprising at least one of the group consisting of an active pharmaceutical ingredient, a bioactive compound or living cells.

4. The composition for additive manufacturing comprising a composite hydrogel, according to the previous claims, **characterized by** the active pharmaceutical ingredient is at least one selected from the group comprising non-steroidal anti-inflammatory drugs (NSAIDs), anti-cancer drugs, and wound healing drugs.

5. The composition for additive manufacturing comprising a composite hydrogel, according to any one of the claims 3 to 4, **characterized by** the bioactive compound is at least one selected from the group consisting of phenolic compounds, including but not limited to flavonoids, isoflavonoids, phenolic acids, cinnamic acids, lignans, coumarins, and curcuminoids, including the salts, esters, glucosides, and stereoisomers thereof.

6. The composition for additive manufacturing comprising a composite hydrogel, according to any one of the previous claims, **characterized by** the cellulose or cellulose derivative-based particles are selected from the group consisting of cellulose, cellulose acetate, methyl cellulose, hydroxypropyl cellulose (HPC), hydroxyethyl cellulose (HEC), or hydroxypropylmethyl cellulose (HPMC) particles, or their mixtures.

7. A process for obtaining an additive manufacturing composition, as defined in any one of the previous claims, **characterized by** comprising the following steps:

   i. Preparing an alginate aqueous solution in a concentration from 1 to 6%, wherein the percentual corresponds to the mass of alginate in relation to the total volume of solvent;
   ii. Preparing of an aqueous suspension of spherical cellulose or cellulose derivative-based particles in a concentration from 1 to 20%, wherein the percentual corresponds to the mass of said spherical cellulose or cellulose derivative-based particles in relation to the mass of said alginate, and wherein the spherical cellulose or cellulose derivative-based particles have a diameter from 0.1 to 2.00 micrometres;
   iii. Mixing the alginate aqueous solution, obtained in step i), with the aqueous suspension of spherical cellulose or cellulose derivative-based particles, obtained in step ii);
   iv. Performing a pre-crosslinking stage of the mixture obtained in the previous step with an aqueous solution comprising cations of a pre-crosslinking salt, wherein the cation of the pre-crosslinking salt is selected from at least one of the group consisting of a divalent or a trivalent cation;

   wherein the steps i) and ii) are carried out in any order.

8. The process for obtaining an additive manufacturing composition, according to the previous claim, **characterized by** the sodium alginate is obtained from brown algae.

9. The process for obtaining an additive manufacturing composition, according to any one of the claims 7 and 8, **characterized by** the spherical cellulose or cellulose derivative-based particles are obtained from cellulose acetate via a dissolution step followed by a regeneration step.

10. The process for obtaining an additive manufacturing composition, according to any one of the claims 7 to 9, **characterized by** the active pharmaceutical ingredient or the bioactive compound are added into the aqueous suspension of spherical cellulose or cellulose derivative-based particles prior to step iii).

11. A process of additive manufacturing of an object, **characterized by** comprising the following steps:

   a) preparation of a composition for additive manufacturing comprising a composite hydrogel, as defined in any one of the claims 1 to 6;
   b) deposition by a system configured for the manufacture of objects by additive manufacturing of a plurality of lines of said composition for additive manufacturing comprising a composite hydrogel over a printing table to form an object;

   wherein the step b) comprises an extrusion step of said composition for additive manufacturing through a nozzle comprised in a head of the system configured for the manufacture of objects by additive manufacturing.

12. The process of additive manufacturing of an object, according to the previous claim, **characterized in that** the object is selected from the group consisting of a biomedical device or a living tissue.

13. The process of additive manufacturing of an object, according to any one of the claims, 11 or 12 **characterized in that** after the step b) is performed a full-crosslinking stage of an additive manufactured object, wherein said additive manufactured object is immersed into an aqueous solution comprising cations of a full-crosslinking salt, wherein the cation of the full-crosslinking salt is selected from at least one of the group consisting of a divalent or a trivalent cation.

**Patentansprüche**

1. Eine Zusammensetzung zur additiven Herstellung, bestehend aus einem Hydrogel-Verbundwerkstoff, **dadurch gekennzeichnet, dass** sie Folgendes umfasst:

   Ein Salz der Alginsäure, das als Alginat bezeichnet wird, in einer Konzentration von 1 bis 6 %, worin der prozentuale Anteil der Masse des Alginats dem Verhältnis zum Volumen der Zusammensetzung entspricht, und kugelförmige Cellulose oder Partikel auf Basis von Cellulosederivaten in einer Konzentration von 1 bis 20 %, worin der prozentuale Anteil der Masse dieser kugelförmigen Cellulose oder Partikel auf Basis von Cellulosederivaten dem Verhältnis zur Masse des Alginats entspricht, und
   ein Kation eines vorvernetzenden Salzes in einer Konzentration von 0,05 bis 0,25 %, worin der prozentuale Anteil der Masse des Kations dem Verhältnis zum Gesamtvolumen der Zusammensetzung entspricht,
   wobei das Kation des Alginats aus mindestens einer, der aus einem Alkalimetall oder einem Erdalkalimetall bestehenden Gruppe ausgewählt wird, und
   wobei die kugelförmige Zellulose oder Partikel auf Basis von Cellulosederivaten einen Durchmesser von 0,1 bis 2,0 Mikrometer aufweisen, und
   wobei das Kation eines vorvernetzenden Salzes aus mindestens einem zweiwertigen oder dreiwertigen Kation ausgewählt wird.

2. Die Zusammensetzung zur additiven Herstellung, bestehend aus einem Hydrogel-Verbundwerkstoff gemäß dem vorhergehenden Anspruch, **dadurch gekennzeichnet, dass** die Zusammensetzung so konfiguriert ist, um in einem additiven Herstellungsprozess extrudiert zu werden.

3. Die Zusammensetzung zur additiven Herstellung, bestehend aus einem Hydrogel-Verbundwerkstoff gemäß einem der vorhergehenden Ansprüche, **dadurch gekennzeichnet, dass** sie ferner mindestens eine der Gruppen umfasst, die aus einem aktiven pharmazeutischen Wirkstoff, einer bioaktiven Verbindung oder lebenden Zellen bestehen.

4. Die Zusammensetzung zur additiven Herstellung, bestehend aus einem Hydrogel-Verbundwerkstoff gemäß den vorhergehenden Ansprüchen, **dadurch gekennzeichnet, dass** der aktive pharmazeutische Wirkstoff mindestens aus der Gruppe ausgewählt wird, die nichtsteroidale entzündungshemmende Medikamente (NSAIDs), Krebsmedikamente und Wundheilmittel umfasst.

5. Die Zusammensetzung zur additiven Herstellung, bestehend aus einem Hydrogel-Verbundwerkstoff gemäß einem der Ansprüche 3 bis 4, **dadurch gekennzeichnet, dass** die bioaktive Verbindung mindestens aus der Gruppe ausgewählt wird, die aus Phenolverbindungen, einschließlich, aber nicht beschränkt auf Flavonoide, Isoflavonoide, Phenolsäuren, Zimtsäuren, Lignanen, Cumarinen und Curcuminoiden, einschließlich ihrer Salze, Ester, Glucoside und Stereoisomere besteht.

6. Die Zusammensetzung zur additiven Herstellung, bestehend aus einem Hydrogel-Verbundwerkstoff gemäß einem der vorhergehenden Ansprüche, **dadurch gekennzeichnet, dass** die auf Cellulose oder Cellulosederivaten basierenden Partikel aus der Gruppe ausgewählt werden, die aus Cellulose, Celluloseacetat, Methylcellulose, Hydroxypropylcellulose (HPC), Hydroxyethylcellulose (HEC) oder Hydroxypropylmethylcellulose (HPMC) Partikeln oder deren Mischungen besteht.

7. Ein Verfahren zum Erhalt einer additiven Herstellungszusammensetzung, wie in einem der vorhergehenden Ansprüche definiert, **dadurch gekennzeichnet, dass** es die folgenden Schritte umfasst:

   i. Herstellung einer wässrigen Alginatlösung in einer Konzentration von 1 bis 6 %, wobei der prozentuale Anteil der Masse des Alginats dem Verhältnis zum Gesamtvolumen des Lösungsmittels entspricht;
   ii Herstellung einer wässrigen Suspension aus kugelförmiger Cellulose oder Partikeln auf Basis von Cellulosederivaten in einer Konzentration von 1 bis 20 %, wobei der prozentuale Anteil der Masse der kugelförmigen Cellulose oder Partikel auf Basis von Cellulosederivaten dem Verhältnis zur Masse des Alginats entspricht und wobei die kugelförmige Cellulose oder Partikel auf Basis von Cellulosederivaten einen Durchmesser von 0,1 bis 2,00 Mikrometer aufweisen;

iii Mischen der in Schritt i) erhaltenen wässrigen Alginatlösung mit der wässrigen Suspension aus kugelförmiger Cellulose oder Partikeln auf Basis von Cellulosederivaten, die in Schritt ii erhalten wurden;

iv. Durchführung einer Vorvernetzungsstufe des im vorhergehenden Schritt erhaltenen Gemisches mit einer wässrigen Lösung, die Kationen eines Vorvernetzungssalzes enthält, wobei das Kation des Vorvernetzungssalzes aus mindestens einer Gruppe ausgewählt wird, die aus einem zweiwertigen oder dreiwertigen Kation besteht,

wobei die Schritte i) und ii in beliebiger Reihenfolge ausgeführt werden.

8. Das Verfahren zum Erhalt einer additiven Herstellungszusammensetzung gemäß dem vorhergehenden Anspruch, **dadurch gekennzeichnet, dass** das Natriumalginat aus Braunalgen gewonnen wird.

9. Das Verfahren zum Erhalt einer additiven Herstellungszusammensetzung gemäß einem der Ansprüche 7 und 8, **dadurch gekennzeichnet, dass** die kugelförmige Cellulose oder Partikel auf Basis von Cellulosederivaten aus Celluloseacetat über einen Auflösungsschritt, gefolgt von einem Regenerationsschritt, erhalten werden.

10. Das Verfahren zum Erhalt einer additiven Herstellungszusammensetzung, gemäß einem der Ansprüche 7 bis 9, **dadurch gekennzeichnet, dass** der aktive pharmazeutische Wirkstoff oder die bioaktive Verbindung vor dem Schritt iii in die wässrige Suspension von kugelförmiger Zellulose- oder Partikel auf Basis von Cellulosederivaten gegeben werden.

11. Ein Verfahren zur additiven Herstellung eines Objekts, **dadurch gekennzeichnet, dass** es die folgenden Schritte umfasst:

a) Herstellung einer Zusammensetzung zur additiven Fertigung, die einen Hydrogel-Verbundwerkstoff gemäß einem der Ansprüche 1 bis 6 umfasst;
b) Abscheidung einer Vielzahl von Linien der genannten Zusammensetzung zur additiven Fertigung, die einen Hydrogel-Verbundwerkstoff über einen Drucktisch durch ein System umfasst, das für die Herstellung von Objekten durch die additive Fertigung konfiguriert ist, um ein Objekt zu bilden;

wobei der Schritt b) einen Extrusionsschritt der Zusammensetzung für die additive Fertigung durch eine Düse umfasst, die in einem Kopf des Systems enthalten ist, das für die Herstellung von Objekten durch additive Fertigung konfiguriert ist.

12. Das Verfahren zur additiven Herstellung eines Objekts gemäß dem vorhergehenden Anspruch, **dadurch gekennzeichnet, dass** das Objekt aus der Gruppe ausgewählt wird, die aus einer biomedizinischen Vorrichtung oder einem lebenden Gewebe besteht.

13. Das Verfahren zur additiven Herstellung eines Objektes gemäß einem der Ansprüche 11 oder 12 **dadurch gekennzeichnet, dass** nach dem Schritt b) eine Vollvernetzungsstufe eines additiv hergestellten Objektes durchgeführt wird, wobei das additiv hergestellte Objekt in eine wässrige Lösung eingetaucht wird, die Kationen eines vollvernetzenden Salzes umfasst, wobei das Kation des vollvernetzenden Salzes aus mindestens einer Gruppe ausgewählt wird, die aus einem zweiwertigen oder einem dreiwertigen Kation besteht.

## Revendications

1. Une composition destinée à une fabrication additive comprenant un hydrogel composite **caractérisé en ce qu'elle** comprend :

un sel d'acide alginique, appelé alginate, dans une concentration de 1 à 6 %, dans laquelle le pourcentage correspond à la masse d'alginate par rapport au volume de ladite composition ; et
des particules sphériques à base de cellulose ou de dérivé de cellulose dans une concentration de 1 à 20 %, dans laquelle le pourcentage correspond à la masse desdites particules sphériques à base de cellulose ou de dérivé de cellulose par rapport à la masse dudit alginate ; et

un cation d'un sel de pré-réticulation dans une concentration de 0,05 à 0,25 %, dans laquelle le pourcentage correspond à la masse dudit cation par rapport au volume total de la composition ;

dans laquelle le cation d'alginate est choisi parmi au moins un élément du groupe constitué d'un métal alcalin ou d'un métal alcalino-terreux ; et

dans laquelle les particules sphériques à base de cellulose ou de dérivé de cellulose ont un diamètre de 0,1 à 2,0 micromètres ; et

dans laquelle le cation d'un sel de pré-réticulation est choisi parmi au moins un d'un cation divalent ou d'un cation trivalent.

2. La composition pour fabrication additive comprenant un hydrogel composite, selon la revendication précédente, **caractérisée en ce que** ladite composition est configurée pour être extrudée dans un procédé de fabrication additive.

3. La composition pour fabrication additive comprenant un hydrogel composite, selon l'une quelconque des revendications précédentes, **caractérisée en ce qu'elle** comprend en outre au moins un élément du groupe constitué d'un ingrédient pharmaceutique actif, d'un composé bioactif ou de cellules vivantes.

4. La composition pour fabrication additive comprenant un hydrogel composite, selon les revendications précédentes, **caractérisée en ce que** l'ingrédient pharmaceutique actif est au moins un élément choisi parmi le groupe comprenant les médicaments anti-inflammatoires non stéroïdiens (AINS), les médicaments anticancéreux et les médicaments cicatrisants.

5. La composition pour fabrication additive comprenant un hydrogel composite, selon l'une quelconque des revendications 3 à 4, **caractérisée en ce que** le composé bioactif est au moins un composé choisi parmi le groupe constitué de composés phénoliques, comprenant, sans s'y limiter, des flavonoïdes, des isoflavonoïdes, des acides phénoliques, des acides cinnamiques, des lignanes, des coumarines et des curcuminoïdes, y compris leurs sels, esters, glucosides et stéréoisomères.

6. La composition pour fabrication additive comprenant un hydrogel composite, selon l'une quelconque des revendications précédentes, **caractérisée en ce que** les particules à base de cellulose ou de dérivé de cellulose sont choisies parmi le groupe constitué de particules de cellulose, d'acétate de cellulose, de méthylcellulose, d'hydroxypropylcellulose (HPC), d'hydroxyéthylcellulose (HEC) ou d'hydroxypropylméthylcellulose (HPMC), ou de leurs mélanges.

7. Un processus d'obtention d'une composition de fabrication additive, telle que définie dans l'une quelconque des revendications précédentes, **caractérisé en ce qu'il** comprend les étapes suivantes :

i. la préparation d'une solution aqueuse d'alginate dans une concentration de 1 à 6 %, dans laquelle le pourcentage correspond à la masse d'alginate par rapport au volume total de solvant ;

ii. la préparation d'une suspension aqueuse de particules sphériques à base de cellulose ou de dérivé de cellulose dans une concentration de 1 à 20 %, dans laquelle le pourcentage correspond à la masse desdites particules sphériques à base de cellulose ou de dérivé de cellulose par rapport à la masse dudit alginate, et dans laquelle les particules sphériques à base de cellulose ou de dérivé de cellulose ont un diamètre de 0,1 à 2,00 micromètres ;

iii. le mélange de la solution aqueuse d'alginate, obtenue à l'étape i), avec la suspension aqueuse de particules sphériques à base de cellulose ou de dérivé de cellulose, obtenue à l'étape ii) ;

iv. la réalisation d'une étape de pré-réticulation du mélange obtenu à l'étape précédente avec une solution aqueuse comprenant des cations d'un sel de pré-réticulation, le cation du sel de pré-réticulation étant choisi parmi au moins un cation du groupe constitué par un cation divalent ou un cation trivalent ;

dans lequel les étapes i) et ii) sont effectuées dans n'importe quel ordre.

8. Le processus d'obtention d'une composition de fabrication additive, selon la revendication précédente, **caractérisé en ce que** l'alginate de sodium est obtenu à partir d'algues brunes.

9. Le processus d'obtention d'une composition de fabrication additive, selon l'une quelconque des revendications 7 et 8, **caractérisé en ce que** les particules sphériques à base de cellulose ou de dérivé de cellulose sont obtenues à partir d'acétate de cellulose par le biais d'une étape de dissolution suivie d'une étape de régénération.

10. Le processus d'obtention d'une composition de fabrication additive, selon l'une quelconque des revendications 7 à 9, **caractérisé en ce que** l'ingrédient pharmaceutique actif ou le composé bioactif sont ajoutés dans la suspension aqueuse de particules sphériques à base de cellulose ou de dérivé de cellulose avant l'étape iii).

11. Un processus de fabrication additive d'un objet, **caractérisé en ce qu'**il comprend les étapes suivantes :

> a) la préparation d'une composition pour fabrication additive comprenant un hydrogel composite, telle que définie dans l'une quelconque des revendications 1 à 6 ;
> b) le dépôt par un système configuré pour la fabrication d'objets par fabrication additive d'une pluralité de lignes de ladite composition pour fabrication additive comprenant un hydrogel composite sur une table d'impression pour former un objet ;

> dans lequel l'étape b) comprend une étape d'extrusion de ladite composition pour fabrication additive à travers une buse comprise dans une tête du système configuré pour la fabrication d'objets par fabrication additive.

12. Le processus de fabrication additive d'un objet, selon la revendication précédente, **caractérisé en ce que** l'objet est choisi parmi le groupe constitué d'un dispositif biomédical ou d'un tissu vivant.

13. Le processus de fabrication additive d'un objet, selon l'une quelconque des revendications 11 ou 12, **caractérisé en ce qu'**après l'étape b) est réalisée une étape de réticulation complète d'un objet fabriqué de manière additive, dans lequel ledit objet fabriqué de manière additive est immergé dans une solution aqueuse comprenant des cations d'un sel de réticulation complète, dans lequel le cation du sel de réticulation complète est choisi parmi au moins un cation du groupe constitué d'un cation divalent ou d'un cation trivalent.

SU-70 4.0kV 11.2mm x5.00k SE(M)          10.0um

SU-70 4.0kV 11.2mm x9.00k SE(M)          5.00um

Fig. 1

Fig. 2

Fig. 3

Fig. 4

Fig5

Fig. 6

Fig. 7

Fig. 8

Fig. 9

Fig. 10

Fig. 11

Fig. 12

Fig. 13

50 kPa
90 kPa
130 kPa
160 kPa
200 kPa

5 mm.s⁻¹
7.5 mm.s⁻¹
10 mm.s⁻¹
12.5 mm.s⁻¹
15.0 mm.s⁻¹

Fig. 14

Fig. 15

Fig. 16

SU-70 4.0kV 9.7mm x30 SE(M)                    1.00mm

SU-70 4.0kV 10.2mm x20.0k SE(M)                2.00um

Fig. 17

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021234141 A1, Guasch Camell Judit
- WO 2019173637 A1, Nelson Kimberly
- WO 2020081982 A1, Ogle Brenda M
- WO 2020245331 A1, Berglund Linn and Oksman Kristiina
- WO 2017210663 A1, Gatenholm Paul

### Non-patent literature cited in the description

- **SCHWAB, A et al.** Printability and Shape Fidelity of Bioinks in 3D Bioprinting. *Chem. Rev*, 2020, vol. 120, 11028-11055
- **UNAGOLLA, J. M** ; **JAYASURIYA, A. C**. Hydrogel-based 3D bioprinting: A comprehensive review on cell-laden hydrogels, bioink formulations, and future perspectives. *Appl. Mater. Today*, 2020, vol. 18, 100479
- **KUMAR, S** ; **THARAYIL, A** ; **THOMAS, S**. 3D Bioprinting of Nature-Inspired Hydrogel Inks Based on Synthetic Polymers. *ACS Appl. Polym. Mater*, 2021, vol. 3, 3685-3701
- Importance of Alginate Bioink for 3D Bioprinting in Tissue Engineering and Regenerative Medicine. **DATTA, S** ; **BARUA, R** ; **DAS, J**. Alginates - Recent Uses of This Natural Polymer. IntechOpen, 2020
- **TAGHIZADEH, M et al.** Chitosan-based inks for 3D printing and bioprinting. *Green Chem*, 2022, vol. 24, 62-101
- **WANG, X et al.** Gelatin-Based Hydrogels for Organ 3D Bioprinting. *Polymers (Basel)*, 2017, vol. 9, 401
- **YOON HYUNG SUN et al.** Cellulose nanocrystals as support nanomaterials for dual droplet-based freeform 3D printing. *CARBOHYDRATE POLYMERS*, 22 July 2021, vol. 272 (118459)
- **NEVES, M. I** ; **MORONI, L** ; **BARRIAS, C. C**. Modulating Alginate Hydrogels for Improved Biological Performance as Cellular 3D Microenvironments. *Front. Bioeng. Biotechnol*, 2020, vol. 8
- **KONG, H. J.** ; **KAIGLER, D** ; **KIM, K** ; **MOONEY, D. J**. Controlling Rigidity and Degradation of Alginate Hydrogels via Molecular Weight Distribution. *Biomacromolecules*, 2004, vol. 5, 1720-1727
- **PAHLEVANZADEH, F et al.** Recent Trends in Three-Dimensional Bioinks Based on Alginate for Biomedical Applications. *Materials (Basel)*, 2020, vol. 13, 3980
- **HAN, C et al.** Effects of nanocellulose on alginate/-gelatin bio-inks for extrusion-based 3D printing. *BioResources*, 2020, vol. 15, 7357-7373
- **NGUYEN, D et al.** Cartilage Tissue Engineering by the 3D Bioprinting of iPS Cells in a Nanocellulose/Alginate. *Bioink. Sci. Rep*, 2017, vol. 7, 658
- **WU, Z et al.** Biocompatibility evaluation of a 3D-bioprinted alginate-GelMA-bacterial nanocellulose (BNC) scaffold laden with oriented-growth RSC96 cells. *Mater. Sci. Eng. C*, 2021, vol. 129, 112393
- **WU, Y** ; **LIN, Z. Y. (WILLIAM** ; **WENGER, A. C** ; **TAM, K. C** ; **TANG, X. (SHIRLEY**. 3D bioprinting of liver-mimetic construct with alginate/cellulose nanocrystal hybrid bioink. *Bioprinting*, 2018, vol. 9, 1-6
- **WANG, X** ; **WANG, Q** ; **XU, C**. Nanocellulose-based inks for 3D bioprinting: Key aspects in research development and challenging perspectives in applications - a mini review. *Bioengineering*, 2020, vol. 7, 40
- **HOSPODIUK, M** ; **DEY, M.** ; **SOSNOSKI, D** ; **OZBOLAT, I. T**. The bioink: A comprehensive review on bioprintable materials. *Biotechnol. Adv*, 2017, vol. 35, 217-239
- **WONDRACZEK, H** ; **PETZOLD-WELCKE, K** ; **FARDIM, P** ; **HEINZE, T**. Nanoparticles from conventional cellulose esters: evaluation of preparation methods. *Cellulose*, 2013, vol. 20, 751-760
- **CARVALHO, J. P. F.** ; **SILVA, A. C. Q.** ; **SILVESTRE, A. J. D.** ; **FREIRE, C. S. R** ; **VILELA, C**. Spherical Cellulose Micro and Nanoparticles: A Review of Recent Developments and Applications. *Nanomaterials*, 2021, vol. 11, 2744
- **WONDRACZEK, H.** ; **PETZOLD-WELCKE, K** ; **FARDIM, P** ; **HEINZE, T**. Nanoparticles from conventional cellulose esters: evaluation of preparation methods. *Cellulose*, 2013, vol. 20, 751-760

- **MOSMANN, T**. Rapid colorimetric assay for cellular growth and survival: application to proliferation and cytotoxicity assays. *J. Immunol. Methods*, 1983, vol. 65, 55-63
- **UNAGOLLA, J. M** ; **JAYASURIYA, A. C**. Hydrogel-based 3D bioprinting: A comprehensive review on cell-laden hydrogels, bioink formulations, and future perspectives. *Appl. Mater*, 2020 (18), 100479
- **NEVES, M. I.** ; **MORONI, L** ; **BARRIAS, C. C**. Modulating Alginate Hydrogels for Improved Biological Performance as Cellular 3D Microenvironments. *Front. Bioeng. Biotechnol*, 2020, vol. 8
- **KONG, H. J** ; **KAIGLER, D** ; **KIM, K** ; **MOONEY, D. J**. Controlling Rigidity and Degradation of Alginate Hydrogels via Molecular Weight Distribution. *Biomacromolecules*, 2004, vol. 5, 1720-1727
- **NGUYEN, D et al.** Cartilage Tissue Engineering by the 3D Bioprinting of iPS Cells in a Nanocellulose/Alginate Bioink. *Sci. Rep*, 2017, vol. 7, 658
- **WU, Z et al.** Biocompatibility evaluation of a 3D-bioprinted alginate-GelMA-bacteria nanocellulose (BNC) scaffold laden with oriented-growth RSC96 cells. *Mater. Sci. Eng. C*, 2021, vol. 129, 112393
- **WU, Y.** ; **LIN, Z. Y. (WILLIAM** ; **WENGER, A. C** ; **TAM, K. C** ; **TANG, X. (SHIRLEY**. 3D bioprinting of liver-mimetic construct with alginate/cellulose nanocrystal hybrid bioink. *Bioprinting*, 2018, vol. 9, 1-6
- **WANG, X.** ; **WANG, Q** ; **XU, C**. Nanocellulose-based inks for 3d bioprinting: Key aspects in research development and challenging perspectives in applications-a mini review. *Bioengineering*, 2020, vol. 7, 40
- **HOSPODIUK, M.** ; **DEY, M** ; **SOSNOSKI, D** ; **OZBOLAT, I. T**. The bioink: A comprehensive review on bioprintable materials. *Biotechnol. Adv*, 2017, vol. 35, 217-239
- **CARVALHO, J. P. F** ; **SILVA, A. C. Q** ; **SILVESTRE, A. J. D** ; **FREIRE, C. S. R** ; **VILELA, C**. Spherical Cellulose Micro and Nanoparticles: A Review of Recent Developments and Applications. *Nanomaterials*, 2021, vol. 11, 2744